# EUROPÄISCHE PATENTANMELDUNG

(11) **EP 1 775 349 A1**
(43) Veröffentlichungstag der Anmeldung: **18.04.2007**
(21) Anmeldenummer: 06090189.9
(22) Anmeldetag: 13.10.2006
(51) Int. Cl.: C12Q 1/68

(54) **Verfahren zur Prognose und Diagnose eines Pilzbefalls an Salatpflanzen**

(30) Priorität: 14.10.2005 DE 102005049751; 02.01.2006 DE 102006001081
(71) Anmelder: Institut für Gemüse- & Zierpflanzenbau e.V., 14979 Grossbeeren (DE)
(72) Erfinder: Grosch, Rita, Dr., 12439 Berlin (DE); Waschke, Astrid, Dipl.-Bio., 12167 Berlin (DE); Jabaji-Hare, Suha H., Ste-Anne-de-Bellevue, Quebec H9X 2E3 (CA); Schneider, Johannes H.M., Dr., 4600 AA Bergen op Zoom (NL); Kofoet, Andreas, Dr., 12139 Berlin (DE)
(74) Vertreter: Boeckh, Tobias

(57) **Zusammenfassung**

Die Erfindung betrifft ein Verfahren zur Diagnose bzw. zur prophylaktischen Bestimmung eines Pilzbefalls an Gemüsepflanzen, insbesondere Salatpflanzen, die Erfindung betrifft auch einen Primer bzw. ein Primerpaar, sowie die Verwendung dieses Primers in dem genannten Verfahren zur Pilzbestimmung; weiterhin betrifft die Erfindung einen Kit und die Verwendung des Kits zur Bestimmung von Pilzbefall.

SEQUENCE LISTING

<110>  Institut für Gemüse- und Zierpflanzenbau e.V.

<120>  Verfahren zur Prognose und Diagnose eines Pilzbefalls an
       Salatpflanzen

<130>  XII 1139-06

<140>  06090189.9
<141>  2006-10-13

<150>  DE 10 2005 049 751.9
<151>  2005-10-14

<150>  DE 10 2006 001 081.7
<151>  2006-01-02

<160>  132

<170>  PatentIn version 3.3

<210>  1
<211>  1547
<212>  DNA
<213>  Rhizoctonia solani virus


<220>
<221>  misc_feature
<223>  Synthetic primer

<400>  1
ggtgaggtca catgataaga aaggttgtcc tattgactcg gaaaaaatac tataattatg      60

agaaaaatga ggggtttcag gtgatgtcta tgacatggca ccagaaccat tgtcacccca     120

gttgtgcaaa actgctccca ccctttctca agcatgttga gtatgggtga ttcatcatag     180

gtctttatat tgcaaaacag ttggttcaaa ccatgtgttt caatgattca ggatatctgc     240

tcatcaacac taggaatgtc atgtggcaag atatgcaggt ccattctcaa caattggctc     300

aatcattctg ctaacgttgg tgacaacact gggtttatat tgaaaacaac atggaacaac     360

tgttccaaat catatttgac aaatttcata tttatacaag atgccaacca actcagctcc     420

tgaacgtttg agacaaggtt tacacatgat ttggtttcag caatctgcca agctccagat     480

ttgcatggct gtgtaggggg gtggggggag cacagcatcc cccagtggct gtgatatact     540

atgtaaatgg atatgcgtgg atatatctat atatacctgg acatgatata tatggtaata     600

acccttccca aagtgggtta tgacactaga gtaggtggta tcaacaagag gtaattgacc     660

tgggtgtacc catggtcaag agacctccta tatattgtac agaggcagtg ttatgtgtaa     720

ttgtatatga aaaactatga ccaataagaa ccctgctcag tctgcagcct tactcatgta     780

gtgttgtaga cgtcatgcac agaggtggca cttataaggc cggtctatac tactacacat     840

ggagtctcta tgctactcta actacaatca gggaggggtt ggaggcaatg ctgggagtga     900

ctatgttagc acgctcaacc atactgcagc tgactcagct atctgtcgtt ggccttgggc     960

tgctatgaag tgtagagtgg tgacaaagag gtaaaagacc tggggattac catggttgat    1020

tgccctcctt atatattcta acaaggcagt gtaattacat atacaatatc aaattctgta    1080

cccaataaga accccgttca gcctgtggcc ttactcatcc tgtgacatgg aggcttggca 1140

ctgactgagg ggatgtgact aaggtggtgg aggctgacca ataagtgctg ggctgttggt 1200

aagcactggg ggttatgtaa caagcactga attcatgtga ctaaaaaact aaagctaagg 1260

tgttttgttt aaattttgag aaaataggaa taaatccttt ggtgtgtgtg tcatgcctac 1320

aacatgtagt gacatggagg tcttggcact gactgatggg gatgtgactt tggtgcttct 1380

gcttgttatt gctgatgtaa taagtgccaa ggtcacatga ttcaaaaagt tgttctattg 1440

acttggaaaa atactatagc ttgagtgcca cctctaggca gcaaagacct tgctcagagg 1500

tggggtgctg tatgtgtttg cttgttcaat gtatattgac ctcacca 1547

<210> 2
<211> 12
<212> DNA
<213> Rhizoctonia solani virus

<220>
<221> misc_feature
<223> Synthetic primer

<400> 2
ggtgaggtca ca 12

<210> 3
<211> 12
<212> DNA
<213> Rhizoctonia solani virus

<220>
<221> misc_feature
<223> Synthetic primer

<400> 3
tgataagaaa gg 12

<210> 4
<211> 12
<212> DNA
<213> Rhizoctonia solani virus

<220>
<221> misc_feature
<223> Synthetic primer

<400> 4
ttgtcctatt ga 12

<210> 5
<211> 12
<212> DNA
<213> Rhizoctonia solani virus

<220>
<221> misc_feature

<223>  Synthetic primer

<400>  5
aactatgacc aa                                                          12


<210>  6
<211>  12
<212>  DNA
<213>  Rhizoctonia solani virus


<220>
<221>  misc_feature
<223>  Synthetic primer

<400>  6
taagaaccct gc                                                          12


<210>  7
<211>  12
<212>  DNA
<213>  Rhizoctonia solani virus


<220>
<221>  misc_feature
<223>  Synthetic primer

<400>  7
tcagtctgca gc                                                          12


<210>  8
<211>  12
<212>  DNA
<213>  Rhizoctonia solani virus


<220>
<221>  misc_feature
<223>  Synthetic primer

<400>  8
cttactcatg ta                                                          12


<210>  9
<211>  12
<212>  DNA
<213>  Rhizoctonia solani virus


<220>
<221>  misc_feature
<223>  Synthetic primer

<400>  9
gtgttgtaga cg                                                          12


<210>  10
<211>  12
<212>  DNA
<213>  Rhizoctonia solani virus

<220>
<221>  misc_feature
<223>  Synthetic primer

<400>  10
tcatgcacag ag                                                          12


<210>  11
<211>  12
<212>  DNA
<213>  Rhizoctonia solani virus


<220>
<221>  misc_feature
<223>  Synthetic primer

<400>  11
gtggcactta ta                                                          12


<210>  12
<211>  12
<212>  DNA
<213>  Rhizoctonia solani virus


<220>
<221>  misc_feature
<223>  Synthetic primer

<400>  12
aggccggtct at                                                          12


<210>  13
<211>  12
<212>  DNA
<213>  Rhizoctonia solani virus


<220>
<221>  misc_feature
<223>  Synthetic primer

<400>  13
actactacac at                                                          12


<210>  14
<211>  12
<212>  DNA
<213>  Rhizoctonia solani virus


<220>
<221>  misc_feature
<223>  Synthetic primer

<400>  14
ggagtctcta tg                                                          12


<210>  15
<211>  12
<212>  DNA
<213>  Rhizoctonia solani virus

<220>
<221>  misc_feature
<223>  Synthetic primer

<400>  15
ctactctaac ta                                                          12


<210>  16
<211>  12
<212>  DNA
<213>  Rhizoctonia solani virus


<220>
<221>  misc_feature
<223>  Synthetic primer

<400>  16
caatcaggga gg                                                          12


<210>  17
<211>  12
<212>  DNA
<213>  Rhizoctonia solani virus


<220>
<221>  misc_feature
<223>  Synthetic primer

<400>  17
ggttggaggc aa                                                          12


<210>  18
<211>  12
<212>  DNA
<213>  Rhizoctonia solani virus


<220>
<221>  misc_feature
<223>  Synthetic primer

<400>  18
tgctgggagt ga                                                          12


<210>  19
<211>  12
<212>  DNA
<213>  Rhizoctonia solani virus


<220>
<221>  misc_feature
<223>  Synthetic primer

<400>  19
ctatgttagc ac                                                          12


<210>  20
<211>  12

<212>  DNA
<213>  Rhizoctonia solani virus

<220>
<221>  misc_feature
<223>  Synthetic primer

<400>  20
gctcaaccat ac                                                          12

<210>  21
<211>  12
<212>  DNA
<213>  Rhizoctonia solani virus

<220>
<221>  misc_feature
<223>  Synthetic primer

<400>  21
tgcagctgac tc                                                          12

<210>  22
<211>  12
<212>  DNA
<213>  Rhizoctonia solani virus

<220>
<221>  misc_feature
<223>  Synthetic primer

<400>  22
agctatctgt cg                                                          12

<210>  23
<211>  12
<212>  DNA
<213>  Rhizoctonia solani virus

<220>
<221>  misc_feature
<223>  Synthetic primer

<400>  23
ttggccttgg gc                                                          12

<210>  24
<211>  12
<212>  DNA
<213>  Rhizoctonia solani virus

<220>
<221>  misc_feature
<223>  Synthetic primer

<400>  24
tgctatgaag tg                                                          12

<210>  25
<211>  12
<212>  DNA
<213>  Rhizoctonia solani virus


<220>
<221>  misc_feature
<223>  Synthetic primer

<400>  25
tagagtggtg ac                                                          12


<210>  26
<211>  12
<212>  DNA
<213>  Rhizoctonia solani virus


<220>
<221>  misc_feature
<223>  Synthetic primer

<400>  26
aaagaggtaa aa                                                          12


<210>  27
<211>  12
<212>  DNA
<213>  Rhizoctonia solani virus


<220>
<221>  misc_feature
<223>  Synthetic primer

<400>  27
gacctgggga tt                                                          12


<210>  28
<211>  12
<212>  DNA
<213>  Rhizoctonia solani virus


<220>
<221>  misc_feature
<223>  Synthetic primer

<400>  28
accatggttg at                                                          12


<210>  29
<211>  12
<212>  DNA
<213>  Rhizoctonia solani virus


<220>
<221>  misc_feature
<223>  Synthetic primer

<400>  29
tgccctcctt at                                                          12

<210>  30
<211>  12
<212>  DNA
<213>  Rhizoctonia solani virus


<220>
<221>  misc_feature
<223>  Synthetic primer

<400>  30
atattctaac aa                                                          12


<210>  31
<211>  12
<212>  DNA
<213>  Rhizoctonia solani virus


<220>
<221>  misc_feature
<223>  Synthetic primer

<400>  31
ggcagtgtaa tt                                                          12


<210>  32
<211>  12
<212>  DNA
<213>  Rhizoctonia solani virus


<220>
<221>  misc_feature
<223>  Synthetic primer

<400>  32
acatatacaa ta                                                          12


<210>  33
<211>  12
<212>  DNA
<213>  Rhizoctonia solani virus


<220>
<221>  misc_feature
<223>  Synthetic primer

<400>  33
tcaaattctg ta                                                          12


<210>  34
<211>  12
<212>  DNA
<213>  Rhizoctonia solani virus


<220>
<221>  misc_feature
<223>  Synthetic primer

<400>  34
cccaataaga ac                                                          12


<210>  35
<211>  12
<212>  DNA
<213>  Rhizoctonia solani virus


<220>
<221>  misc_feature
<223>  Synthetic primer

<400>  35
cccgttcagc ct                                                          12


<210>  36
<211>  12
<212>  DNA
<213>  Rhizoctonia solani virus


<220>
<221>  misc_feature
<223>  Synthetic primer

<400>  36
gtggccttac tc                                                          12


<210>  37
<211>  12
<212>  DNA
<213>  Rhizoctonia solani virus


<220>
<221>  misc_feature
<223>  Synthetic primer

<400>  37
atcctgtgac at                                                          12


<210>  38
<211>  12
<212>  DNA
<213>  Rhizoctonia solani virus


<220>
<221>  misc_feature
<223>  Synthetic primer

<400>  38
ggaggcttgg ca                                                          12


<210>  39
<211>  12
<212>  DNA
<213>  Rhizoctonia solani virus


<220>
<221>  misc_feature

<223>  Synthetic primer

<400>  39
ctgactgagg gg                                                          12


<210>  40
<211>  12
<212>  DNA
<213>  Rhizoctonia solani virus


<220>
<221>  misc_feature
<223>  Synthetic primer

<400>  40
atgtgactaa gg                                                          12


<210>  41
<211>  12
<212>  DNA
<213>  Rhizoctonia solani virus


<220>
<221>  misc_feature
<223>  Synthetic primer

<400>  41
tggtggaggc tg                                                          12


<210>  42
<211>  12
<212>  DNA
<213>  Rhizoctonia solani virus


<220>
<221>  misc_feature
<223>  Synthetic primer

<400>  42
accaataagt gc                                                          12


<210>  43
<211>  12
<212>  DNA
<213>  Rhizoctonia solani virus


<220>
<221>  misc_feature
<223>  Synthetic primer

<400>  43
tgggctgttg gt                                                          12


<210>  44
<211>  12
<212>  DNA
<213>  Rhizoctonia solani virus

<220>
<221>  misc_feature
<223>  Synthetic primer

<400>  44
aagcactggg gg                                                          12


<210>  45
<211>  12
<212>  DNA
<213>  Rhizoctonia solani virus


<220>
<221>  misc_feature
<223>  Synthetic primer

<400>  45
ttatgtaaca ag                                                          12


<210>  46
<211>  12
<212>  DNA
<213>  Rhizoctonia solani virus


<220>
<221>  misc_feature
<223>  Synthetic primer

<400>  46
cactgaattc at                                                          12


<210>  47
<211>  12
<212>  DNA
<213>  Rhizoctonia solani virus


<220>
<221>  misc_feature
<223>  Synthetic primer

<400>  47
gtgactaaaa aa                                                          12


<210>  48
<211>  12
<212>  DNA
<213>  Rhizoctonia solani virus


<220>
<221>  misc_feature
<223>  Synthetic primer

<400>  48
ctaaagctaa gg                                                          12


<210>  49
<211>  12
<212>  DNA
<213>  Rhizoctonia solani virus

<220>
<221>  misc_feature
<223>  Synthetic primer

<400>  49
tgttttgttt aa                                                          12


<210>  50
<211>  12
<212>  DNA
<213>  Rhizoctonia solani virus


<220>
<221>  misc_feature
<223>  Synthetic primer

<400>  50
attttgagaa aa                                                          12


<210>  51
<211>  12
<212>  DNA
<213>  Rhizoctonia solani virus


<220>
<221>  misc_feature
<223>  Synthetic primer

<400>  51
taggaataaa tc                                                          12


<210>  52
<211>  9
<212>  DNA
<213>  Rhizoctonia solani virus


<220>
<221>  misc_feature
<223>  Synthetic primer

<400>  52
ctttggtgt                                                               9


<210>  53
<211>  12
<212>  DNA
<213>  Rhizoctonia solani virus


<220>
<221>  misc_feature
<223>  Synthetic primer

<400>  53
atagagactc ca                                                          12


<210>  54
<211>  12

<212>  DNA
<213>  Rhizoctonia solani virus

<220>
<221>  misc_feature
<223>  Synthetic primer

<400>  54
tgtgtagtag ta                                                          12

<210>  55
<211>  12
<212>  DNA
<213>  Rhizoctonia solani virus

<220>
<221>  misc_feature
<223>  Synthetic primer

<400>  55
tagaccggcc tt                                                          12

<210>  56
<211>  12
<212>  DNA
<213>  Rhizoctonia solani virus

<220>
<221>  misc_feature
<223>  Synthetic primer

<400>  56
ataagtgcca cc                                                          12

<210>  57
<211>  12
<212>  DNA
<213>  Rhizoctonia solani virus

<220>
<221>  misc_feature
<223>  Synthetic primer

<400>  57
tctgtgcatg ac                                                          12

<210>  58
<211>  12
<212>  DNA
<213>  Rhizoctonia solani virus

<220>
<221>  misc_feature
<223>  Synthetic primer

<400>  58
gtctacaaca ct                                                          12

<210>  59
<211>  12
<212>  DNA
<213>  Rhizoctonia solani virus


<220>
<221>  misc_feature
<223>  Synthetic primer

<400>  59
acatgagtaa gg                                                          12


<210>  60
<211>  12
<212>  DNA
<213>  Rhizoctonia solani virus


<220>
<221>  misc_feature
<223>  Synthetic primer

<400>  60
ctgcagactg ag                                                          12


<210>  61
<211>  12
<212>  DNA
<213>  Rhizoctonia solani virus


<220>
<221>  misc_feature
<223>  Synthetic primer

<400>  61
cagggttctt at                                                          12


<210>  62
<211>  12
<212>  DNA
<213>  Rhizoctonia solani virus


<220>
<221>  misc_feature
<223>  Synthetic primer

<400>  62
tggtcatagt tt                                                          12


<210>  63
<211>  12
<212>  DNA
<213>  Rhizoctonia solani virus


<220>
<221>  misc_feature
<223>  Synthetic primer

<400>  63
ttcatataca at                                                          12

<210>  64
<211>  12
<212>  DNA
<213>  Rhizoctonia solani virus

<220>
<221>  misc_feature
<223>  Synthetic primer

<400>  64
tacacataac ac                                                          12

<210>  65
<211>  12
<212>  DNA
<213>  Rhizoctonia solani virus

<220>
<221>  misc_feature
<223>  Synthetic primer

<400>  65
tgcctctgta ca                                                          12

<210>  66
<211>  12
<212>  DNA
<213>  Rhizoctonia solani virus

<220>
<221>  misc_feature
<223>  Synthetic primer

<400>  66
atatataagg ag                                                          12

<210>  67
<211>  12
<212>  DNA
<213>  Rhizoctonia solani virus

<220>
<221>  misc_feature
<223>  Synthetic primer

<400>  67
gtctcttgac ca                                                          12

<210>  68
<211>  12
<212>  DNA
<213>  Rhizoctonia solani virus

<220>
<221>  misc_feature
<223>  Synthetic primer

<400>  68
tgggtacacc ca                                                          12


<210>  69
<211>  12
<212>  DNA
<213>  Rhizoctonia solani virus


<220>
<221>  misc_feature
<223>  Synthetic primer

<400>  69
ggtcaattac ct                                                          12


<210>  70
<211>  12
<212>  DNA
<213>  Rhizoctonia solani virus


<220>
<221>  misc_feature
<223>  Synthetic primer

<400>  70
cttgttgata cc                                                          12


<210>  71
<211>  12
<212>  DNA
<213>  Rhizoctonia solani virus


<220>
<221>  misc_feature
<223>  Synthetic primer

<400>  71
acctactcta gt                                                          12


<210>  72
<211>  12
<212>  DNA
<213>  Rhizoctonia solani virus


<220>
<221>  misc_feature
<223>  Synthetic primer

<400>  72
gtcataaccc ac                                                          12


<210>  73
<211>  12
<212>  DNA
<213>  Rhizoctonia solani virus


<220>
<221>  misc_feature

<223>  Synthetic primer

<400>  73
tttgggaagg gt                                                          12


<210>  74
<211>  12
<212>  DNA
<213>  Rhizoctonia solani virus


<220>
<221>  misc_feature
<223>  Synthetic primer

<400>  74
tattaccata ta                                                          12


<210>  75
<211>  12
<212>  DNA
<213>  Rhizoctonia solani virus


<220>
<221>  misc_feature
<223>  Synthetic primer

<400>  75
tatcatgtcc ag                                                          12


<210>  76
<211>  12
<212>  DNA
<213>  Rhizoctonia solani virus


<220>
<221>  misc_feature
<223>  Synthetic primer

<400>  76
gtatatatag at                                                          12


<210>  77
<211>  12
<212>  DNA
<213>  Rhizoctonia solani virus


<220>
<221>  misc_feature
<223>  Synthetic primer

<400>  77
atatccacgc at                                                          12


<210>  78
<211>  12
<212>  DNA
<213>  Rhizoctonia solani virus

<220>
<221>  misc_feature
<223>  Synthetic primer

<400>  78
atccatttac at                                                          12


<210>  79
<211>  12
<212>  DNA
<213>  Rhizoctonia solani virus


<220>
<221>  misc_feature
<223>  Synthetic primer

<400>  79
agtatatcac ag                                                          12


<210>  80
<211>  12
<212>  DNA
<213>  Rhizoctonia solani virus


<220>
<221>  misc_feature
<223>  Synthetic primer

<400>  80
ccactggggg at                                                          12


<210>  81
<211>  12
<212>  DNA
<213>  Rhizoctonia solani virus


<220>
<221>  misc_feature
<223>  Synthetic primer

<400>  81
gctgtgctcc cc                                                          12


<210>  82
<211>  12
<212>  DNA
<213>  Rhizoctonia solani virus


<220>
<221>  misc_feature
<223>  Synthetic primer

<400>  82
ccacccccct ac                                                          12


<210>  83
<211>  12
<212>  DNA
<213>  Rhizoctonia solani virus

<220>
<221>  misc_feature
<223>  Synthetic primer

<400>  83
acagccatgc aa                                                          12


<210>  84
<211>  12
<212>  DNA
<213>  Rhizoctonia solani virus


<220>
<221>  misc_feature
<223>  Synthetic primer

<400>  84
atctggagct tg                                                          12


<210>  85
<211>  12
<212>  DNA
<213>  Rhizoctonia solani virus


<220>
<221>  misc_feature
<223>  Synthetic primer

<400>  85
gcagattgct ga                                                          12


<210>  86
<211>  12
<212>  DNA
<213>  Rhizoctonia solani virus


<220>
<221>  misc_feature
<223>  Synthetic primer

<400>  86
aaccaaatca tg                                                          12


<210>  87
<211>  12
<212>  DNA
<213>  Rhizoctonia solani virus


<220>
<221>  misc_feature
<223>  Synthetic primer

<400>  87
tgtaaacctt gt                                                          12


<210>  88
<211>  12

<212>  DNA
<213>  Rhizoctonia solani virus

<220>
<221>  misc_feature
<223>  Synthetic primer

<400>  88
ctcaaacgtt ca                                                          12

<210>  89
<211>  12
<212>  DNA
<213>  Rhizoctonia solani virus

<220>
<221>  misc_feature
<223>  Synthetic primer

<400>  89
ggagctgagt tg                                                          12

<210>  90
<211>  12
<212>  DNA
<213>  Rhizoctonia solani virus

<220>
<221>  misc_feature
<223>  Synthetic primer

<400>  90
gttggcatct tg                                                          12

<210>  91
<211>  12
<212>  DNA
<213>  Rhizoctonia solani virus

<220>
<221>  misc_feature
<223>  Synthetic primer

<400>  91
tataagtatg aa                                                          12

<210>  92
<211>  12
<212>  DNA
<213>  Rhizoctonia solani virus

<220>
<221>  misc_feature
<223>  Synthetic primer

<400>  92
atttgtcaaa ta                                                          12

<210>  93
<211>  12
<212>  DNA
<213>  Rhizoctonia solani virus


<220>
<221>  misc_feature
<223>  Synthetic primer

<400>  93
tgatttggaa ca                                                          12


<210>  94
<211>  12
<212>  DNA
<213>  Rhizoctonia solani virus


<220>
<221>  misc_feature
<223>  Synthetic primer

<400>  94
gttgttccat gt                                                          12


<210>  95
<211>  12
<212>  DNA
<213>  Rhizoctonia solani virus


<220>
<221>  misc_feature
<223>  Synthetic primer

<400>  95
tgttttcaat at                                                          12


<210>  96
<211>  12
<212>  DNA
<213>  Rhizoctonia solani virus


<220>
<221>  misc_feature
<223>  Synthetic primer

<400>  96
aaacccagtg tt                                                          12


<210>  97
<211>  12
<212>  DNA
<213>  Rhizoctonia solani virus


<220>
<221>  misc_feature
<223>  Synthetic primer

<400>  97
gtcaccaacg tt                                                          12

<210>  98
<211>  12
<212>  DNA
<213>  Rhizoctonia solani virus


<220>
<221>  misc_feature
<223>  Synthetic primer

<400>  98
agcagaatga tt                                                          12


<210>  99
<211>  12
<212>  DNA
<213>  Rhizoctonia solani virus


<220>
<221>  misc_feature
<223>  Synthetic primer

<400>  99
gagccaattg tt                                                          12


<210>  100
<211>  12
<212>  DNA
<213>  Rhizoctonia solani virus


<220>
<221>  misc_feature
<223>  Synthetic primer

<400>  100
gagaatggac ct                                                          12


<210>  101
<211>  12
<212>  DNA
<213>  Rhizoctonia solani virus


<220>
<221>  misc_feature
<223>  Synthetic primer

<400>  101
gcatatcttg cc                                                          12


<210>  102
<211>  12
<212>  DNA
<213>  Rhizoctonia solani virus


<220>
<221>  misc_feature
<223>  Synthetic primer

<400>  102
acatgacatt cc                                                          12


<210>  103
<211>  12
<212>  DNA
<213>  Rhizoctonia solani virus


<220>
<221>  misc_feature
<223>  Synthetic primer

<400>  103
tagtgttgat ga                                                          12


<210>  104
<211>  12
<212>  DNA
<213>  Rhizoctonia solani virus


<220>
<221>  misc_feature
<223>  Synthetic primer

<400>  104
gcagatatcc tg                                                          12


<210>  105
<211>  12
<212>  DNA
<213>  Rhizoctonia solani virus


<220>
<221>  misc_feature
<223>  Synthetic primer

<400>  105
aatcattgaa ac                                                          12


<210>  106
<211>  12
<212>  DNA
<213>  Rhizoctonia solani virus


<220>
<221>  misc_feature
<223>  Synthetic primer

<400>  106
acatgggttg aa                                                          12


<210>  107
<211>  12
<212>  DNA
<213>  Rhizoctonia solani virus


<220>
<221>  misc_feature

<223>  Synthetic primer

<400>  107
acaactgttt tg                                                          12


<210>  108
<211>  12
<212>  DNA
<213>  Rhizoctonia solani virus


<220>
<221>  misc_feature
<223>  Synthetic primer

<400>  108
catataagac ct                                                          12


<210>  109
<211>  12
<212>  DNA
<213>  Rhizoctonia solani virus


<220>
<221>  misc_feature
<223>  Synthetic primer

<400>  109
atgatgatca cc                                                          12


<210>  110
<211>  8
<212>  DNA
<213>  Rhizoctonia solani virus


<220>
<221>  misc_feature
<223>  Synthetic primer

<400>  110
atactcac                                                                8


<210>  111
<211>  21
<212>  DNA
<213>  Rhizoctonia solani virus


<220>
<221>  misc_feature
<223>  Synthetic primer

<400>  111
agcgtgctaa catagtcact c                                                21


<210>  112
<211>  21
<212>  DNA
<213>  Rhizoctonia solani virus

<220>
<221>  misc_feature
<223>  Synthetic primer

<400>  112
acactagagt aggtggtatc a                                                21


<210>  113
<211>  12
<212>  DNA
<213>  Rhizoctonia solani virus

<400>  113
catggaggtc tt                                                          12


<210>  114
<211>  12
<212>  DNA
<213>  Rhizoctonia solani virus

<400>  114
ggcactgact ga                                                          12


<210>  115
<211>  12
<212>  DNA
<213>  Rhizoctonia solani virus

<400>  115
tggggatgtg ac                                                          12


<210>  116
<211>  12
<212>  DNA
<213>  Rhizoctonia solani virus

<400>  116
tttggtgctt ct                                                          12


<210>  117
<211>  12
<212>  DNA
<213>  Rhizoctonia solani virus

<400>  117
gcttgttatt gc                                                          12


<210>  118
<211>  12
<212>  DNA
<213>  Rhizoctonia solani virus

<400>  118
tgatgtaata ag                                                          12


<210>  119
<211>  12
<212>  DNA
<213>  Rhizoctonia solani virus

<400>  119

tgccaaggtc ac 12

<210> 120
<211> 12
<212> DNA
<213> Rhizoctonia solani virus

<400> 120
atgattcaaa aa 12

<210> 121
<211> 12
<212> DNA
<213> Rhizoctonia solani virus

<400> 121
gttgttctat tg 12

<210> 122
<211> 12
<212> DNA
<213> Rhizoctonia solani virus

<400> 122
acttggaaaa at 12

<210> 123
<211> 12
<212> DNA
<213> Rhizoctonia solani virus

<400> 123
actatagctt ga 12

<210> 124
<211> 12
<212> DNA
<213> Rhizoctonia solani virus

<400> 124
gtgccacctc ta 12

<210> 125
<211> 12
<212> DNA
<213> Rhizoctonia solani virus

<400> 125
ggcagcaaag ac 12

<210> 126
<211> 12
<212> DNA
<213> Rhizoctonia solani virus

<400> 126
cttgctcaga gg 12

<210> 127
<211> 12

<212>  DNA
<213>  Rhizoctonia solani virus

<400>  127
tggggtgctg ta                                                          12


<210>  128
<211>  12
<212>  DNA
<213>  Rhizoctonia solani virus

<400>  128
tgtgtttgct tg                                                          12


<210>  129
<211>  12
<212>  DNA
<213>  Rhizoctonia solani virus

<400>  129
ttcaatgtat at                                                          12


<210>  130
<211>  11
<212>  DNA
<213>  Rhizoctonia solani virus

<400>  130
tgacctcacc a                                                           11


<210>  131
<211>  21
<212>  DNA
<213>  Rhizoctonia solani virus

<400>  131
agcgtgctaa catagtcact c                                                21


<210>  132
<211>  21
<212>  DNA
<213>  Rhizoctonia solani virus

<400>  132
acactagagt aggtggtatc a                                                21

SEQUENCE LISTING

<110>  Institut für Gemüse- und Zierpflanzenbau e.V.

<120>  Verfahren zur Prognose und Diagnose eines Pilzbefalls an
       Salatpflanzen

<130>  XII 1139-06

<140>  06090189.9
<141>  2006-10-13

<150>  DE 10 2005 049 751.9
<151>  2005-10-14

<150>  DE 10 2006 001 081.7
<151>  2006-01-02

<160>  132

<170>  PatentIn version 3.3

<210>  1
<211>  1547
<212>  DNA
<213>  Rhizoctonia solani virus

<400>  1
ggtgaggtca catgataaga aaggttgtcc tattgactcg gaaaaaatac tataattatg      60

agaaaaatga ggggtttcag gtgatgtcta tgacatggca ccagaaccat tgtcacccca     120

gttgtgcaaa actgctccca ccctttctca agcatgttga gtatgggtga ttcatcatag     180

gtctttatat tgcaaaacag ttggttcaaa ccatgtgttt caatgattca ggatatctgc     240

tcatcaacac taggaatgtc atgtggcaag atatgcaggt ccattctcaa caattggctc     300

aatcattctg ctaacgttgg tgacaacact gggtttatat tgaaaacaac atggaacaac     360

tgttccaaat catatttgac aaatttcata tttatacaag atgccaacca actcagctcc     420

tgaacgtttg agacaaggtt tacacatgat ttggtttcag caatctgcca agctccagat     480

ttgcatggct gtgtaggggg gtggggggag cacagcatcc cccagtggct gtgatatact     540

atgtaaatgg atatgcgtgg atatatctat atatacctgg acatgatata tatggtaata     600

acccttccca aagtgggtta tgacactaga gtaggtggta tcaacaagag gtaattgacc     660

tgggtgtacc catggtcaag agacctccta tatattgtac agaggcagtg ttatgtgtaa     720

ttgtatatga aaaactatga ccaataagaa ccctgctcag tctgcagcct tactcatgta     780

gtgttgtaga cgtcatgcac agaggtggca cttataaggc cggtctatac tactacacat     840

ggagtctcta tgctactcta actacaatca gggaggggtt ggaggcaatg ctgggagtga     900

ctatgttagc acgctcaacc atactgcagc tgactcagct atctgtcgtt ggccttgggc     960

tgctatgaag tgtagagtgg tgacaaagag gtaaaagacc tggggattac catggttgat    1020

tgccctcctt atatattcta acaaggcagt gtaattacat atacaatatc aaattctgta    1080

cccaataaga accccgttca gcctgtggcc ttactcatcc tgtgacatgg aggcttggca    1140

ctgactgagg ggatgtgact aaggtggtgg aggctgacca ataagtgctg ggctgttggt    1200

aagcactggg ggttatgtaa caagcactga attcatgtga ctaaaaaact aaagctaagg 1260

tgttttgttt aaattttgag aaaataggaa taaatccttt ggtgtgtgtg tcatgcctac 1320

aacatgtagt gacatggagg tcttggcact gactgatggg gatgtgactt tggtgcttct 1380

gcttgttatt gctgatgtaa taagtgccaa ggtcacatga ttcaaaaagt tgttctattg 1440

acttggaaaa atactatagc ttgagtgcca cctctaggca gcaaagacct tgctcagagg 1500

tggggtgctg tatgtgtttg cttgttcaat gtatattgac ctcacca 1547

<210> 2
<211> 12
<212> DNA
<213> Rhizoctonia solani virus

<220>
<221> misc_feature
<223> Synthetic primer

<220>
<221> misc_feature
<223> Synthetic primer position 1-12 of SEQ ID No. 1

<400> 2
ggtgaggtca ca 12

<210> 3
<211> 12
<212> DNA
<213> Rhizoctonia solani virus

<220>
<221> misc_feature
<223> Synthetic primer position 13-24 of SEQ ID No. 1

<400> 3
tgataagaaa gg 12

<210> 4
<211> 12
<212> DNA
<213> Rhizoctonia solani virus

<220>
<221> misc_feature
<223> Synthetic primer position 25-36 of SEQ ID No. 1

<400> 4
ttgtcctatt ga 12

<210> 5
<211> 12
<212> DNA
<213> Rhizoctonia solani virus

<220>
<221> misc_feature
<223> Synthetic primer position 37-48 of SEQ ID No. 1

<400>  5
aactatgacc aa                                                          12


<210>  6
<211>  12
<212>  DNA
<213>  Rhizoctonia solani virus


<220>
<221>  misc_feature
<223>  Synthetic primer  position 49-60 of SEQ ID No. 1

<400>  6
taagaaccct gc                                                          12


<210>  7
<211>  12
<212>  DNA
<213>  Rhizoctonia solani virus


<220>
<221>  misc_feature
<223>  Synthetic primer position 61-72 of SEQ ID No. 1

<400>  7
tcagtctgca gc                                                          12


<210>  8
<211>  12
<212>  DNA
<213>  Rhizoctonia solani virus


<220>
<221>  misc_feature
<223>  Synthetic primer position 73-84 of SEQ ID No. 1

<400>  8
cttactcatg ta                                                          12


<210>  9
<211>  12
<212>  DNA
<213>  Rhizoctonia solani virus


<220>
<221>  misc_feature
<223>  Synthetic primer position 85-96 of SEQ ID No. 1

<400>  9
gtgttgtaga cg                                                          12


<210>  10
<211>  12
<212>  DNA
<213>  Rhizoctonia solani virus


<220>

<221>  misc_feature
<223>  Synthetic primer position 97-108 of SEQ ID No. 1

<400>  10
tcatgcacag ag                                                          12


<210>  11
<211>  12
<212>  DNA
<213>  Rhizoctonia solani virus


<220>
<221>  misc_feature
<223>  Synthetic primer position 109-120 of SEQ ID No. 1

<400>  11
gtggcactta ta                                                          12


<210>  12
<211>  12
<212>  DNA
<213>  Rhizoctonia solani virus


<220>
<221>  misc_feature
<223>  Synthetic primer position 121-132 of SEQ ID No. 1

<400>  12
aggccggtct at                                                          12


<210>  13
<211>  12
<212>  DNA
<213>  Rhizoctonia solani virus


<220>
<221>  misc_feature
<223>  Synthetic primer position 133-144 of SEQ ID No. 1

<400>  13
actactacac at                                                          12


<210>  14
<211>  12
<212>  DNA
<213>  Rhizoctonia solani virus


<220>
<221>  misc_feature
<223>  Synthetic primer position 145-156 of SEQ ID No. 1

<400>  14
ggagtctcta tg                                                          12


<210>  15
<211>  12
<212>  DNA
<213>  Rhizoctonia solani virus

<220>
<221>  misc_feature
<223>  Synthetic primer position 157-168 of SEQ ID No. 1

<400>  15
ctactctaac ta                                                          12


<210>  16
<211>  12
<212>  DNA
<213>  Rhizoctonia solani virus


<220>
<221>  misc_feature
<223>  Synthetic primer position 169-180 of SEQ ID No. 1

<400>  16
caatcaggga gg                                                          12


<210>  17
<211>  12
<212>  DNA
<213>  Rhizoctonia solani virus


<220>
<221>  misc_feature
<223>  Synthetic primer position 181-192 of SEQ ID No. 1

<400>  17
ggttggaggc aa                                                          12


<210>  18
<211>  12
<212>  DNA
<213>  Rhizoctonia solani virus


<220>
<221>  misc_feature
<223>  Synthetic primer position 193-204 of SEQ ID No. 1

<400>  18
tgctgggagt ga                                                          12


<210>  19
<211>  12
<212>  DNA
<213>  Rhizoctonia solani virus


<220>
<221>  misc_feature
<223>  Synthetic primer position 205-216 of SEQ ID No. 1

<400>  19
ctatgttagc ac                                                          12


<210>  20
<211>  12
<212>  DNA

<213>  Rhizoctonia solani virus

<220>
<221>  misc_feature
<223>  Synthetic primer position 217-228 of SEQ ID No. 1

<400>  20
gctcaaccat ac                                                          12

<210>  21
<211>  12
<212>  DNA
<213>  Rhizoctonia solani virus

<220>
<221>  misc_feature
<223>  Synthetic primer position 229-240 of SEQ ID No. 1

<400>  21
tgcagctgac tc                                                          12

<210>  22
<211>  12
<212>  DNA
<213>  Rhizoctonia solani virus

<220>
<221>  misc_feature
<223>  Synthetic primer position 241-252 of SEQ ID No. 1

<400>  22
agctatctgt cg                                                          12

<210>  23
<211>  12
<212>  DNA
<213>  Rhizoctonia solani virus

<220>
<221>  misc_feature
<223>  Synthetic primer position 253-264 of SEQ ID No. 1

<400>  23
ttggccttgg gc                                                          12

<210>  24
<211>  12
<212>  DNA
<213>  Rhizoctonia solani virus

<220>
<221>  misc_feature
<223>  Synthetic primer position 265-276 of SEQ ID No. 1

<400>  24
tgctatgaag tg                                                          12

<210>  25

<211>  12
<212>  DNA
<213>  Rhizoctonia solani virus


<220>
<221>  misc_feature
<223>  Synthetic primer position 277-288 of SEQ ID No. 1

<400>  25
tagagtggtg ac                                                          12


<210>  26
<211>  12
<212>  DNA
<213>  Rhizoctonia solani virus


<220>
<221>  misc_feature
<223>  Synthetic primer position 289-300 of SEQ ID No. 1

<400>  26
aaagaggtaa aa                                                          12


<210>  27
<211>  12
<212>  DNA
<213>  Rhizoctonia solani virus


<220>
<221>  misc_feature
<223>  Synthetic primer position 301-312 of SEQ ID No. 1

<400>  27
gacctgggga tt                                                          12


<210>  28
<211>  12
<212>  DNA
<213>  Rhizoctonia solani virus


<220>
<221>  misc_feature
<223>  Synthetic primer position 313-324 of SEQ ID No. 1

<400>  28
accatggttg at                                                          12


<210>  29
<211>  12
<212>  DNA
<213>  Rhizoctonia solani virus


<220>
<221>  misc_feature
<223>  Synthetic primer position 325-336 of SEQ ID No. 1

<400>  29
tgccctcctt at                                                          12

<210>  30
<211>  12
<212>  DNA
<213>  Rhizoctonia solani virus


<220>
<221>  misc_feature
<223>  Synthetic primer position 337-348 of SEQ ID No. 1

<400>  30
atattctaac aa                                                          12


<210>  31
<211>  12
<212>  DNA
<213>  Rhizoctonia solani virus


<220>
<221>  misc_feature
<223>  Synthetic primer position 349-360 of SEQ ID No. 1

<400>  31
ggcagtgtaa tt                                                          12


<210>  32
<211>  12
<212>  DNA
<213>  Rhizoctonia solani virus


<220>
<221>  misc_feature
<223>  Synthetic primer position 361-372 of SEQ ID No. 1

<400>  32
acatatacaa ta                                                          12


<210>  33
<211>  12
<212>  DNA
<213>  Rhizoctonia solani virus


<220>
<221>  misc_feature
<223>  Synthetic primer position 373-384 of SEQ ID No. 1

<400>  33
tcaaattctg ta                                                          12


<210>  34
<211>  12
<212>  DNA
<213>  Rhizoctonia solani virus


<220>
<221>  misc_feature
<223>  Synthetic primer position 385-396 of SEQ ID No. 1

<400>  34

cccaataaga ac                                                          12

<210>  35
<211>  12
<212>  DNA
<213>  Rhizoctonia solani virus

<220>
<221>  misc_feature
<223>  Synthetic primer position 397-408 of SEQ ID No. 1

<400>  35
cccgttcagc ct                                                          12

<210>  36
<211>  12
<212>  DNA
<213>  Rhizoctonia solani virus

<220>
<221>  misc_feature
<223>  Synthetic primer position 409-420 of SEQ ID No. 1

<400>  36
gtggccttac tc                                                          12

<210>  37
<211>  12
<212>  DNA
<213>  Rhizoctonia solani virus

<220>
<221>  misc_feature
<223>  Synthetic primer position 421-432 of SEQ ID No. 1

<400>  37
atcctgtgac at                                                          12

<210>  38
<211>  12
<212>  DNA
<213>  Rhizoctonia solani virus

<220>
<221>  misc_feature
<223>  Synthetic primer position 433-444 of SEQ ID No. 1

<400>  38
ggaggcttgg ca                                                          12

<210>  39
<211>  12
<212>  DNA
<213>  Rhizoctonia solani virus

<220>
<221>  misc_feature
<223>  Synthetic primer position 445-456 of SEQ ID No. 1

<400>  39
ctgactgagg gg                                                          12

<210>  40
<211>  12
<212>  DNA
<213>  Rhizoctonia solani virus

<220>
<221>  misc_feature
<223>  Synthetic primer position 457-468 of SEQ ID No. 1

<400>  40
atgtgactaa gg                                                          12

<210>  41
<211>  12
<212>  DNA
<213>  Rhizoctonia solani virus

<220>
<221>  misc_feature
<223>  Synthetic primer position 469-480 of SEQ ID No. 1

<400>  41
tggtggaggc tg                                                          12

<210>  42
<211>  12
<212>  DNA
<213>  Rhizoctonia solani virus

<220>
<221>  misc_feature
<223>  Synthetic primer position 481-492 of SEQ ID No. 1

<400>  42
accaataagt gc                                                          12

<210>  43
<211>  12
<212>  DNA
<213>  Rhizoctonia solani virus

<220>
<221>  misc_feature
<223>  Synthetic primer position 493-504 of SEQ ID No. 1

<400>  43
tgggctgttg gt                                                          12

<210>  44
<211>  12
<212>  DNA
<213>  Rhizoctonia solani virus

<220>

<221>  misc_feature
<223>  Synthetic primer position 505-516 of SEQ ID No. 1

<400>  44
aagcactggg gg                                                          12


<210>  45
<211>  12
<212>  DNA
<213>  Rhizoctonia solani virus


<220>
<221>  misc_feature
<223>  Synthetic primer position 517-528 of SEQ ID No. 1

<400>  45
ttatgtaaca ag                                                          12


<210>  46
<211>  12
<212>  DNA
<213>  Rhizoctonia solani virus


<220>
<221>  misc_feature
<223>  Synthetic primer position 529-540 of SEQ ID No. 1

<400>  46
cactgaattc at                                                          12


<210>  47
<211>  12
<212>  DNA
<213>  Rhizoctonia solani virus


<220>
<221>  misc_feature
<223>  Synthetic primer position 541-552 of SEQ ID No. 1

<400>  47
gtgactaaaa aa                                                          12


<210>  48
<211>  12
<212>  DNA
<213>  Rhizoctonia solani virus


<220>
<221>  misc_feature
<223>  Synthetic primer position 553-564 of SEQ ID No. 1

<400>  48
ctaaagctaa gg                                                          12


<210>  49
<211>  12
<212>  DNA
<213>  Rhizoctonia solani virus

<220>
<221>  misc_feature
<223>  Synthetic primer position 565-576 of SEQ ID No. 1

<400>  49
tgttttgttt aa                                                          12


<210>  50
<211>  12
<212>  DNA
<213>  Rhizoctonia solani virus


<220>
<221>  misc_feature
<223>  Synthetic primer position 577-588 of SEQ ID No. 1

<400>  50
attttgagaa aa                                                          12


<210>  51
<211>  12
<212>  DNA
<213>  Rhizoctonia solani virus


<220>
<221>  misc_feature
<223>  Synthetic primer position 589-600 of SEQ ID No. 1

<400>  51
taggaataaa tc                                                          12


<210>  52
<211>  9
<212>  DNA
<213>  Rhizoctonia solani virus


<220>
<221>  misc_feature
<223>  Synthetic primer position 601-612 of SEQ ID No. 1

<400>  52
ctttggtgt                                                               9


<210>  53
<211>  12
<212>  DNA
<213>  Rhizoctonia solani virus


<220>
<221>  misc_feature
<223>  Synthetic primer position 613-624 of SEQ ID No. 1

<400>  53
atagagactc ca                                                          12


<210>  54
<211>  12
<212>  DNA

<213>  Rhizoctonia solani virus

<220>
<221>  misc_feature
<223>  Synthetic primer position 625-636 of SEQ ID No. 1

<400>  54
tgtgtagtag ta                                                          12

<210>  55
<211>  12
<212>  DNA
<213>  Rhizoctonia solani virus

<220>
<221>  misc_feature
<223>  Synthetic primer position 637-648 of SEQ ID No. 1

<400>  55
tagaccggcc tt                                                          12

<210>  56
<211>  12
<212>  DNA
<213>  Rhizoctonia solani virus

<220>
<221>  misc_feature
<223>  Synthetic primer position 649-660 of SEQ ID No. 1

<400>  56
ataagtgcca cc                                                          12

<210>  57
<211>  12
<212>  DNA
<213>  Rhizoctonia solani virus

<220>
<221>  misc_feature
<223>  Synthetic primer position 661-672 of SEQ ID No. 1

<400>  57
tctgtgcatg ac                                                          12

<210>  58
<211>  12
<212>  DNA
<213>  Rhizoctonia solani virus

<220>
<221>  misc_feature
<223>  Synthetic primer position 673-684 of SEQ ID No. 1

<400>  58
gtctacaaca ct                                                          12

<210>  59

<211>  12
<212>  DNA
<213>  Rhizoctonia solani virus

<220>
<221>  misc_feature
<223>  Synthetic primer position 685-696 of SEQ ID No. 1

<400>  59
acatgagtaa gg                                                          12

<210>  60
<211>  12
<212>  DNA
<213>  Rhizoctonia solani virus

<220>
<221>  misc_feature
<223>  Synthetic primer position 697-708 of SEQ ID No. 1

<400>  60
ctgcagactg ag                                                          12

<210>  61
<211>  12
<212>  DNA
<213>  Rhizoctonia solani virus

<220>
<221>  misc_feature
<223>  Synthetic primer position 709-720 of SEQ ID No. 1

<400>  61
cagggttctt at                                                          12

<210>  62
<211>  12
<212>  DNA
<213>  Rhizoctonia solani virus

<220>
<221>  misc_feature
<223>  Synthetic primer position 721-732 of SEQ ID No. 1

<400>  62
tggtcatagt tt                                                          12

<210>  63
<211>  12
<212>  DNA
<213>  Rhizoctonia solani virus

<220>
<221>  misc_feature
<223>  Synthetic primer position 733-744 of SEQ ID No. 1

<400>  63
ttcatataca at                                                          12

<210> 64
<211> 12
<212> DNA
<213> Rhizoctonia solani virus

<220>
<221> misc_feature
<223> Synthetic primer position 745-756 of SEQ ID No. 1

<400> 64
tacacataac ac 12

<210> 65
<211> 12
<212> DNA
<213> Rhizoctonia solani virus

<220>
<221> misc_feature
<223> Synthetic primer position 757-768 of SEQ ID No. 1

<400> 65
tgcctctgta ca 12

<210> 66
<211> 12
<212> DNA
<213> Rhizoctonia solani virus

<220>
<221> misc_feature
<223> Synthetic primer position 769-780 of SEQ ID No. 1

<400> 66
atatataagg ag 12

<210> 67
<211> 12
<212> DNA
<213> Rhizoctonia solani virus

<220>
<221> misc_feature
<223> Synthetic primer position 781-792 of SEQ ID No. 1

<400> 67
gtctcttgac ca 12

<210> 68
<211> 12
<212> DNA
<213> Rhizoctonia solani virus

<220>
<221> misc_feature
<223> Synthetic primer position 793-804 of SEQ ID No. 1

<400> 68

tgggtacacc ca                                                          12

<210>  69
<211>  12
<212>  DNA
<213>  Rhizoctonia solani virus

<220>
<221>  misc_feature
<223>  Synthetic primer position 805-816 of SEQ ID No. 1

<400>  69
ggtcaattac ct                                                          12

<210>  70
<211>  12
<212>  DNA
<213>  Rhizoctonia solani virus

<220>
<221>  misc_feature
<223>  Synthetic primer position 817-828 of SEQ ID No. 1

<400>  70
cttgttgata cc                                                          12

<210>  71
<211>  12
<212>  DNA
<213>  Rhizoctonia solani virus

<220>
<221>  misc_feature
<223>  Synthetic primer position 829-840 of SEQ ID No. 1

<400>  71
acctactcta gt                                                          12

<210>  72
<211>  12
<212>  DNA
<213>  Rhizoctonia solani virus

<220>
<221>  misc_feature
<223>  Synthetic primer position 841-852 of SEQ ID No. 1

<400>  72
gtcataaccc ac                                                          12

<210>  73
<211>  12
<212>  DNA
<213>  Rhizoctonia solani virus

<220>
<221>  misc_feature
<223>  Synthetic primer position 853-864 of SEQ ID No. 1

<400>  73
tttgggaagg gt                                                          12


<210>  74
<211>  12
<212>  DNA
<213>  Rhizoctonia solani virus


<220>
<221>  misc_feature
<223>  Synthetic primer position 865-876 of SEQ ID No. 1

<400>  74
tattaccata ta                                                          12


<210>  75
<211>  12
<212>  DNA
<213>  Rhizoctonia solani virus


<220>
<221>  misc_feature
<223>  Synthetic primer position 877-888 of SEQ ID No. 1

<400>  75
tatcatgtcc ag                                                          12


<210>  76
<211>  12
<212>  DNA
<213>  Rhizoctonia solani virus


<220>
<221>  misc_feature
<223>  Synthetic primer position 889-900 of SEQ ID No. 1

<400>  76
gtatatatag at                                                          12


<210>  77
<211>  12
<212>  DNA
<213>  Rhizoctonia solani virus


<220>
<221>  misc_feature
<223>  Synthetic primer position 901-912 of SEQ ID No. 1

<400>  77
atatccacgc at                                                          12


<210>  78
<211>  12
<212>  DNA
<213>  Rhizoctonia solani virus


<220>

<221>  misc_feature
<223>  Synthetic primer position 913-924 of SEQ ID No. 1

<400>  78
atccatttac at                                                          12


<210>  79
<211>  12
<212>  DNA
<213>  Rhizoctonia solani virus


<220>
<221>  misc_feature
<223>  Synthetic primer position 925-936 of SEQ ID No. 1

<400>  79
agtatatcac ag                                                          12


<210>  80
<211>  12
<212>  DNA
<213>  Rhizoctonia solani virus


<220>
<221>  misc_feature
<223>  Synthetic primer position 937-948 of SEQ ID No. 1

<400>  80
ccactggggg at                                                          12


<210>  81
<211>  12
<212>  DNA
<213>  Rhizoctonia solani virus


<220>
<221>  misc_feature
<223>  Synthetic primer position 949-960 of SEQ ID No. 1

<400>  81
gctgtgctcc cc                                                          12


<210>  82
<211>  12
<212>  DNA
<213>  Rhizoctonia solani virus


<220>
<221>  misc_feature
<223>  Synthetic primer position 961-972 of SEQ ID No. 1

<400>  82
   ccacccccct ac                                                          12


<210>  83
<211>  12
<212>  DNA
<213>  Rhizoctonia solani virus

<220>
<221>  misc_feature
<223>  Synthetic primer position 973-984 of SEQ ID No. 1

<400>  83
acagccatgc aa                                                          12


<210>  84
<211>  12
<212>  DNA
<213>  Rhizoctonia solani virus


<220>
<221>  misc_feature
<223>  Synthetic primer position 985-996 of SEQ ID No. 1

<400>  84
atctggagct tg                                                          12


<210>  85
<211>  12
<212>  DNA
<213>  Rhizoctonia solani virus


<220>
<221>  misc_feature
<223>  Synthetic primer position 997-1008 of SEQ ID No. 1

<400>  85
gcagattgct ga                                                          12


<210>  86
<211>  12
<212>  DNA
<213>  Rhizoctonia solani virus


<220>
<221>  misc_feature
<223>  Synthetic primer position 1009-1020 of SEQ ID No. 1

<400>  86
aaccaaatca tg                                                          12


<210>  87
<211>  12
<212>  DNA
<213>  Rhizoctonia solani virus


<220>
<221>  misc_feature
<223>  Synthetic primer position 1021-1032 of SEQ ID No. 1

<400>  87
tgtaaacctt gt                                                          12


<210>  88
<211>  12
<212>  DNA

<213>  Rhizoctonia solani virus

<220>
<221>  misc_feature
<223>  Synthetic primer position 1033-1044 of SEQ ID No. 1

<400>  88
ctcaaacgtt ca                                                          12

<210>  89
<211>  12
<212>  DNA
<213>  Rhizoctonia solani virus

<220>
<221>  misc_feature
<223>  Synthetic primer position 1045-1056 of SEQ ID No. 1

<400>  89
ggagctgagt tg                                                          12

<210>  90
<211>  12
<212>  DNA
<213>  Rhizoctonia solani virus

<220>
<221>  misc_feature
<223>  Synthetic primer position 1057-1068 of SEQ ID No. 1

<400>  90
gttggcatct tg                                                          12

<210>  91
<211>  12
<212>  DNA
<213>  Rhizoctonia solani virus

<220>
<221>  misc_feature
<223>  Synthetic primer position 1069-1080 of SEQ ID No. 1

<400>  91
tataagtatg aa                                                          12

<210>  92
<211>  12
<212>  DNA
<213>  Rhizoctonia solani virus

<220>
<221>  misc_feature
<223>  Synthetic primer position 1081-1092 of SEQ ID No. 1

<400>  92
atttgtcaaa ta                                                          12

<210>  93

<211>  12
<212>  DNA
<213>  Rhizoctonia solani virus

<220>
<221>  misc_feature
<223>  Synthetic primer position 1093-1104 of SEQ ID No. 1

<400>  93
tgatttggaa ca                                                          12

<210>  94
<211>  12
<212>  DNA
<213>  Rhizoctonia solani virus

<220>
<221>  misc_feature
<223>  Synthetic primer position 1105-1116 of SEQ ID No. 1

<400>  94
gttgttccat gt                                                          12

<210>  95
<211>  12
<212>  DNA
<213>  Rhizoctonia solani virus

<220>
<221>  misc_feature
<223>  Synthetic primer position 1117-1128 of SEQ ID No. 1

<400>  95
tgttttcaat at                                                          12

<210>  96
<211>  12
<212>  DNA
<213>  Rhizoctonia solani virus

<220>
<221>  misc_feature
<223>  Synthetic primer position 1129-1140 of SEQ ID No. 1

<400>  96
aaacccagtg tt                                                          12

<210>  97
<211>  12
<212>  DNA
<213>  Rhizoctonia solani virus

<220>
<221>  misc_feature
<223>  Synthetic primer position 1141-1152 of SEQ ID No. 1

<400>  97
gtcaccaacg tt                                                          12

<210>  98
<211>  12
<212>  DNA
<213>  Rhizoctonia solani virus

<220>
<221>  misc_feature
<223>  Synthetic primer position 1153-1164 of SEQ ID No. 1

<400>  98
agcagaatga tt                                                          12

<210>  99
<211>  12
<212>  DNA
<213>  Rhizoctonia solani virus

<220>
<221>  misc_feature
<223>  Synthetic primer position 1165-1176 of SEQ ID No. 1

<400>  99
gagccaattg tt                                                          12

<210>  100
<211>  12
<212>  DNA
<213>  Rhizoctonia solani virus

<220>
<221>  misc_feature
<223>  Synthetic primer position 1177-1188 of SEQ ID No. 1

<400>  100
gagaatggac ct                                                          12

<210>  101
<211>  12
<212>  DNA
<213>  Rhizoctonia solani virus

<220>
<221>  misc_feature
<223>  Synthetic primer position 1189-1200 of SEQ ID No. 1

<400>  101
gcatatcttg cc                                                          12

<210>  102
<211>  12
<212>  DNA
<213>  Rhizoctonia solani virus

<220>
<221>  misc_feature
<223>  Synthetic primer position 1201-1212 of SEQ ID No. 1

<400>  102

acatgacatt cc                                                          12

<210>  103
<211>  12
<212>  DNA
<213>  Rhizoctonia solani virus

<220>
<221>  misc_feature
<223>  Synthetic primer position 1213-1224 of SEQ ID No. 1

<400>  103
tagtgttgat ga                                                          12

<210>  104
<211>  12
<212>  DNA
<213>  Rhizoctonia solani virus

<220>
<221>  misc_feature
<223>  Synthetic primer position 1225-1236 of SEQ ID No. 1

<400>  104
gcagatatcc tg                                                          12

<210>  105
<211>  12
<212>  DNA
<213>  Rhizoctonia solani virus

<220>
<221>  misc_feature
<223>  Synthetic primer position 1237-1248 of SEQ ID No. 1

<400>  105
aatcattgaa ac                                                          12

<210>  106
<211>  12
<212>  DNA
<213>  Rhizoctonia solani virus

<220>
<221>  misc_feature
<223>  Synthetic primer position 1249-1260 of SEQ ID No. 1

<400>  106
acatgggttg aa                                                          12

<210>  107
<211>  12
<212>  DNA
<213>  Rhizoctonia solani virus

<220>
<221>  misc_feature
<223>  Synthetic primer position 1261-1272 of SEQ ID No. 1

<400>  107
acaactgttt tg                                                          12


<210>  108
<211>  12
<212>  DNA
<213>  Rhizoctonia solani virus


<220>
<221>  misc_feature
<223>  Synthetic primer position 1273-1284 of SEQ ID No. 1

<400>  108
catataagac ct                                                          12


<210>  109
<211>  12
<212>  DNA
<213>  Rhizoctonia solani virus


<220>
<221>  misc_feature
<223>  Synthetic primer position 1285-1296 of SEQ ID No. 1

<400>  109
atgatgatca cc                                                          12


<210>  110
<211>  8
<212>  DNA
<213>  Rhizoctonia solani virus


<220>
<221>  misc_feature
<223>  Synthetic primer position 1297-1308 of SEQ ID No. 1

<400>  110
atactcac                                                                8


<210>  111
<211>  21
<212>  DNA
<213>  Rhizoctonia solani virus


<220>
<221>  misc_feature
<223>  Synthetic primer position 1309-1320 of SEQ ID No. 1

<220>
<221>  misc_feature
<223>  Synthetic primer position 1319-1330 of SEQ ID No. 1

<400>  111
agcgtgctaa catagtcact c                                                21


<210>  112
<211>  21
<212>  DNA

<213>  Rhizoctonia solani virus

<220>
<221>  misc_feature
<223>  Synthetic primer position 1321-1332 of SEQ ID No. 1

<400>  112
acactagagt aggtggtatc a                                                21

<210>  113
<211>  12
<212>  DNA
<213>  Rhizoctonia solani virus

<220>
<221>  misc_feature
<223>  Synthetic primer position 1333-1344 of SEQ ID No. 1

<400>  113
catggaggtc tt                                                          12

<210>  114
<211>  12
<212>  DNA
<213>  Rhizoctonia solani virus

<220>
<221>  misc_feature
<223>  Synthetic primer position 1345-1356 of SEQ ID No. 1

<400>  114
ggcactgact ga                                                          12

<210>  115
<211>  12
<212>  DNA
<213>  Rhizoctonia solani virus

<220>
<221>  misc_feature
<223>  Synthetic primer position 1357-1368 of SEQ ID No. 1

<400>  115
tggggatgtg ac                                                          12

<210>  116
<211>  12
<212>  DNA
<213>  Rhizoctonia solani virus

<220>
<221>  misc_feature
<223>  Synthetic primer position 1369-1380 of SEQ ID No. 1

<400>  116
tttggtgctt ct                                                          12

<210>  117

<211>  12
<212>  DNA
<213>  Rhizoctonia solani virus

<220>
<221>  misc_feature
<223>  Synthetic primer position 1381-1392 of SEQ ID No. 1

<400>  117
gcttgttatt gc                                                          12

<210>  118
<211>  12
<212>  DNA
<213>  Rhizoctonia solani virus

<220>
<221>  misc_feature
<223>  Synthetic primer position 1393-1404 of SEQ ID No. 1

<400>  118
tgatgtaata ag                                                          12

<210>  119
<211>  12
<212>  DNA
<213>  Rhizoctonia solani virus

<220>
<221>  misc_feature
<223>  Synthetic primer position 1405-1416 of SEQ ID No. 1

<400>  119
tgccaaggtc ac                                                          12

<210>  120
<211>  12
<212>  DNA
<213>  Rhizoctonia solani virus

<220>
<221>  misc_feature
<223>  Synthetic primer position 1417-1428 of SEQ ID No. 1

<400>  120
atgattcaaa aa                                                          12

<210>  121
<211>  12
<212>  DNA
<213>  Rhizoctonia solani virus

<220>
<221>  misc_feature
<223>  Synthetic primer position 1429-1440 of SEQ ID No. 1

<400>  121
gttgttctat tg                                                          12

<210>  122
<211>  12
<212>  DNA
<213>  Rhizoctonia solani virus

<220>
<221>  misc_feature
<223>  Synthetic primer position 1441-1452 of SEQ ID No. 1

<400>  122
acttggaaaa at                                                          12

<210>  123
<211>  12
<212>  DNA
<213>  Rhizoctonia solani virus

<220>
<221>  misc_feature
<223>  Synthetic primer position 1453-1464 of SEQ ID No. 1

<400>  123
actatagctt ga                                                          12

<210>  124
<211>  12
<212>  DNA
<213>  Rhizoctonia solani virus

<220>
<221>  misc_feature
<223>  Synthetic primer position 1465-1476 of SEQ ID No. 1

<400>  124
gtgccacctc ta                                                          12

<210>  125
<211>  12
<212>  DNA
<213>  Rhizoctonia solani virus

<220>
<221>  misc_feature
<223>  Synthetic primer position 1477-1488 of SEQ ID No. 1

<400>  125
ggcagcaaag ac                                                          12

<210>  126
<211>  12
<212>  DNA
<213>  Rhizoctonia solani virus

<220>
<221>  misc_feature
<223>  Synthetic primer position 1489-1500 of SEQ ID No. 1

<400>  126

cttgctcaga gg                                                          12

<210>  127
<211>  12
<212>  DNA
<213>  Rhizoctonia solani virus

<220>
<221>  misc_feature
<223>  Synthetic primer position 1501-1512 of SEQ ID No. 1

<400>  127
tggggtgctg ta                                                          12

<210>  128
<211>  12
<212>  DNA
<213>  Rhizoctonia solani virus

<220>
<221>  misc_feature
<223>  Synthetic primer position 1513-1524 of SEQ ID No. 1

<400>  128
tgtgtttgct tg                                                          12

<210>  129
<211>  12
<212>  DNA
<213>  Rhizoctonia solani virus

<220>
<221>  misc_feature
<223>  Synthetic primer position 1525-1536 of SEQ ID No. 1

<400>  129
ttcaatgtat at                                                          12

<210>  130
<211>  11
<212>  DNA
<213>  Rhizoctonia solani virus

<220>
<221>  misc_feature
<223>  Synthetic primer position 1537-1547 of SEQ ID No. 1

<400>  130
tgacctcacc a                                                           11

<210>  131
<211>  21
<212>  DNA
<213>  Rhizoctonia solani virus

<220>
<221>  misc_feature
<223>  Synthetic primer

<400>  131
agcgtgctaa catagtcact c                                                21

<210>  132
<211>  21
<212>  DNA
<213>  Rhizoctonia solani virus

<220>
<221>  misc_feature
<223>  Synthetic primer

<400>  132
acactagagt aggtggtatc a                                                21

## Beschreibung

Die Erfindung betrifft ein Verfahren zur Diagnose bzw. zur prophylaktischen Bestimmung eines Pilzbefalls an Gemüsepflanzen, insbesondere Salatpflanzen, die Erfindung betrifft auch einen Primer bzw. ein Primerpaar, sowie die Verwendung dieses Primers in dem genannten Verfahren zur Pilzbestimmung; weiterhin betrifft die Erfindung einen Kit und die Verwendung des Kits zur Bestimmung von Pilzbefall.

Pilzbefall an Pflanzen verursacht jedes Jahr einen Schaden in Millionenhöhe; beispielsweise durch den echten Mehltau, den Sternrußtau, die Blattbräune oder andere Erkrankungen. Besonders für Pflanzen, die nur eine geringe Wuchshöhe erreichen, kann aufgrund ihrer Bodennähe Pilzbefall ein besonders schwieriges Problem darstellen. So ist beispielsweise die durch Pilze verursachte Salatfäule eine weit verbreitete Erkrankung verschiedener Kopfsalatsorten

Durch die Salatfäule vergilben die Blätter des Salates, sie welken und faulen vom Blattstiel her, wodurch die Salatblätter wirtschaftlich nicht mehr verwertet werden können. An den Faulstellen erscheint schneeweißer oder mausgrauer Pilzrasen. Sofern es durch diese optischen Charakteristika nicht möglich ist, die Salatfäule am befallenen Salat zu bestimmen, ist es aktuell zur Diagnose erforderlich, aus den befallenen Pflanzen den Pilz zu kultivieren. Gemäß den geänderten Verbraucherinteressen und den daraufhin modifizierten Vorschriften für Pflanzenschutz seitens der nationalen Regierungen geht es zunehmend um vorbeugende Maßnahmen, die Verhütung der Einschleppung von schädlichen Pilzen oder deren Bekämpfung. Dabei muss zunehmend stärker beachtet werden, dass durch den Umgang mit Antipilzmitteln oder anderen Pflanzenschutzmitteln die Gesundheit von Menschen, Tieren und der Natur nicht gefährdet wird. Am Beispiel des Salats bedeutet dies, einer Kontamination mit Pilzen vorzubeugen, indem nicht mehrere Jahre hintereinander Salat auf der gleichen Fläche angebaut, sondern eine Fruchtfolge eingehalten wird. Weiterhin darf der Salat nicht zu tief eingepflanzt werden, um die Infektionsgefahr aus dem Boden zu vermindern; sollte eine stickstoffbetonte Düngung vermieden werden. Die bekannten biotechnologischen bzw. chemischen Nachweisverfahren für die pflanzenschädigenden Pilze sind sehr aufwändig. Die bisherigen Verfahren erlauben auch nur eine Detektion an bereits befallenen Pflanzen. Es ist nicht möglich, vor der Vegetationsperiode bzw. vor dem Einpflanzen oder dem Sähen der Nutzpflanzen die Bodenbeschaffenheit dahingehend zu untersuchen, ob pflanzen-pathogene Pilze in einer kritischen Konzentration vorhanden sind.

Da gentechnische bzw. molekularbiologische Methoden zu störanfällig waren, erfolgte bisher der Nachweis der pathogenen Pilze durch die Kultivierung auf einem Agarmedium. Durch mikroskopische Beobachtung konnte eine Zuordnung zu einzelnen Untergruppen der Pilze erfolgen. Dieses Verfahren ist sehr aufwändig und erfordert eine jahrelange Erfahrung des mikroskopisch beobachtenden Fachmanns. PCR-Methoden waren, praktisch zum Nachweis aufgrund des hohen zeitlichen Aufwandes, für die entsprechenden Anwendergruppen nicht sinnvoll einsetzbar. Insbesondere war es nicht möglich aus Pflanzen- oder Bodenproben genomische DNA direkt nachzuweisen.

Der direkte Nachweis aus Pflanzen- oder Bodenproben war insbesondere wegen fehlender bzw. mangelhafter Primer nicht möglich. Zwar werden in den US 6,828,097 und US 6,485,907 verschiedene Sequenzen offenbart, die jedoch für den Einsatz der erfindungsgemäß bevorzugten Pflanzenpathogene nicht einsetzbar sind. So wird beispielsweise in der US 6,485,907 ein Primer gegen ITS-Sequenzen aus Rhizoctonia cerealis offenbart, mit dem allerdings keine spezifischen Nachweise gegen das genannte Pflanzenpathogen möglich sind. Dies liegt daran, dass die ITS-Sequenz sehr konserviert ist und demgemäß nur eine geringe Heterogenität zu anderen Gruppen von Pflanzenpathogenen aufweist, so dass mit dem entsprechenden Primer immer zahlreiche Spezies nachgewiesen werden und keinesfalls nur die zur Gruppe von Rhizoctonia cerealis gehörenden. Die US 6,828,097 offenbart ebenfalls verschiedene Sequenzen, die allerdings aufgrund ihrer Größe nicht sinnvoll als Primer eingesetzt werden können. Die US 6,828,097 gibt dem Fachmann auch keine Anregung, aus den offenbarten Sequenzen Sequenzbereiche auszuwählen, die genügend Abstand von den bevorzugten offenbarten Bereichen aufweisen und wobei die ausgewählten Bereiche eine gezielte Auswahl - aufgrund von neuen überraschenden Eigenschaften - darstellen würden. Die bekannten Primer sind jedoch nicht nur bei dem spezifischen Nachweis von einem ausgewählten Pflanzenpathogen nachteilig, sondern sie sind auch nicht so ausgebildet, dass sie den Nachweis von ausgewählten Pflanzenpathogenen in nur einem Arbeitsschritt ermöglichen.

Aufgabe der Erfindung war es daher, Mittel und Verfahren bereitzustellen, die die genannten Nachteile nicht aufweisen und eine einfache, sichere und effiziente Detektion von pflanzen-pathogenen Pilzen bei Nutzpflanzen, insbesondere bei Salaten, ermöglichen. Aufgabe war es auch, Mittel und Verfahren bereitzustellen, die einen spezifischen Nachweis - bevorzugt in einem Arbeitsschritt - von Pflanzenpathogenen ermöglichen, bevorzugt von Salatfäule-Pathogenen, besonders bevorzugt von Rhizoctonia solani Spezies, insbesondere von Rhizoctonia solani AG1-IB.

Diese Aufgabe wird gelöst durch Mittel und Verfahren, die den direkten Nachweis von einer bestimmten genetischen Untergruppe von *Rhizoctonia solani* bevorzugt in einem Arbeitsschritt ermöglichen. Es handelt sich hierbei insbesondere um die Untergruppe IB der Anastomosengruppe 1 (AG1-IB). Diese spezielle Untergruppe ist vor allem als Erreger der Salatfäule bekannt. Die Erfindung richtet sich daher insbesondere auf ein isoliertes Nukleinsäuremolekül, das als Primer verwendet werden kann, der es erlaubt, spezifisch die Untergruppe *R. solani* AG1-IB in nur einem Arbeitsschritt, durch eine PCR zu identifizieren, wobei das isolierte Nukleinsäuremolekül ausgewählt ist aus einer Gruppe bestehend aus:
a) einem Nukleinsäuremolekül kodierend eine Nukleinsäuresequenz gemäß bzw. bestehend aus SEQ ID Nr. 1 bzw. gemäß SEQ ID Nr. 2 bis SEQ ID Nr. 132 oder deren komplementären Nukleotidsequenzen,
b) einem Nukleinsäuremolekül, welches mit einer Nukleinsäuresequenz gemäß a) unter stringenten Bedingungen hybridisiert,
c) einem Nukleinsäuremolekül umfassend eine Nukleotidsequenz, die eine ausreichende Homologie aufweist, um mit einer Nukleotidsequenz gemäß a) oder b) funktionsanalog zu sein,
d) einem Nukleinsäuremolekül, das in Folge des genetischen Codes zu einer Nukleotidsequenz gemäß a) - c) degeneriert ist und
e) einem Nukleinsäuremolekül gemäß einer Nukleotidsequenz nach a) - d), welches durch Deletionen, Additionen, Substitutionen, Translokationen, Inversionen und/oder Insertionen modifiziert und funktionsanalog zu einer Nukleotidsequenz gemäß a) - d) ist bzw. ein Bruchstück einer solchen Sequenz ist.

Die Homologen bzw. Deletions-, Additions-, Substitutions-, Translokations-, Inversions- oder Insertions-Varianten sind hierbei solche modifizierten Nukleinsäuremoleküle, die funktionsanalog zu der Sequenz SEQ ID Nr. 1 sind bzw. zu den erfindungsgemäßen Sequenzen SEQ ID Nr. 2 bis SEQ ID Nr. 132, bevorzugt SEQ ID Nr. 131 und/oder SEQ ID Nr. 132 gehören. D. h. Varianten bzw. Homologe im Sinne der Erfindung sind solche Moleküle, die es als Primer erlauben, spezifisch die Untergruppe *R. solani* AG1-IB in nur einem Arbeitsschritt durch eine PCR zu identifizieren. D. h. der Fachmann mit durchschnittlichem Können wird über die Bedeutung der Begriffe der Homolgen, der Bruchstücke bzw. der durch Deletion, Addition, Substitution, Translokation, Inversion und/oder Insertion modifizierten Nukleotidsequenzen nicht im Unklaren gelassen, da er durch einfache Routineversuche ermitteln kann, ob die bereitgestellten Varianten und Homologen zur Lösung der erfindungsgemäßen Aufgabe eingesetzt werden können. Der Fachmann muss hierzu nicht selbst erfinderisch tätig werden, da die Varianten und Homologen ausreichend - d. h. nacharbeitbar - offenbart sind.

Die Begriffe Deletions-, Additions-, Substitutions-, Translokations-, Inversions-, Insertions-Varianten oder Homologe bzw. Funktionsanaloge sind als Synonyme dergestalt aufzufassen, dass es sich bevorzugt um Äquivalente handelt, die zwar verschieden generiert werden können - z. B. durch Deletions-, Additions-, Substitutions-, Translokations-, Inversions-, oder Insertionsmutationen -, aber im wesentlichen dieselbe Funktion auf im wesentlichen demselben Weg erfüllen und im wesentlichen dasselbe Ereignis hervorbringen, wie die Sequenzen SEQ ID Nr. 1 bis SEQ ID Nr. 132, wobei es für einen Durchschnittsfachmann offensichtlich ist, dass das, was mittels der Sequenzen SEQ ID Nr. 1 bis SEQ ID Nr. 132 erreicht werden kann, auch mittels der Homologen, Funktionsanalogen oder der oben genannten Varianten erreicht werden kann.

Die genannten Begriffe sind daher ausreichend klar und weiterhin wird ausgeschlossen, dass die Mittel, die unter Schutz gestellt werden sollen, über das zu erreichende Ergebnis definiert werden. Die Frage, ob die Homologen, Funktionsanalogen oder Varianten von den Ansprüchen der anmeldungsgemäßen Lehre bzw. von der anmeldungsgemäßen Lehre gemäß der Beschreibung erfasst, wird demgemäß danach beantwortet, ob die Homologen, Funktionsanalogen oder Varianten das der Erfindung zugrunde liegende Problem zwar mit abgewandelten, aber objektiv gleichwirkenden Mitteln lösen und ob der Fachmann durch seine Fachkenntnisse befähigt war, die abgewandelten Mittel (Homologe, Funktionsanaloge, Varianten) als gleichwirkend aufzufinden, wobei die Überlegung, die der Fachmann hierzu anstellen müsste, sich derart am Sinngehalt der in den Patentansprüchen unter Schutz gestellten Lehre orientieren, dass der Fachmann die Verwendung der Homologen, Funktionsanaloge und Varianten als eine Lösung der erfindungsgemäßen Aufgabe in Betracht zieht, die der Lösung der Aufgabe mit den Sequenzen gemäß SEQ ID Nr. 1 bis SEQ ID Nr. 132 gleichwertig ist.

Anmeldungsgemäße Homologen, Funktionsanaloge und Varianten sind demgemäß nur solche, die der Fachmann als gleichwirkend auffinden würde, wobei der Fachmann hierzu Überlegungen anstellen muss, die sich nur am Sinngehalt der im Patentanspruch unter Schutz gestellten Lehre orientieren, wobei der Fachmann die Homologen, Funktionsanalogen und Varianten als eine Lösung in Betracht zieht, die der Lösung gemäß den erfindungsgemäß beanspruchten Sequenzen SEQ ID Nr. 1 bis SEQ ID Nr. 132 gleichwertig ist.

Die Begriffe Homologe, Funktionsanaloge, Varianten sowie "im wesentlichen dieselbe Funktion", "auf im wesentlichen demselben Weg" und "im wesentlichen dasselbe Ergebnis" sind keine relativen Begriffe, da die betreffenden Ausdrücke auf dem betreffenden Fachgebiet der Biologie eine allgemein anerkannte Bedeutung haben. Da die Begriffe Homologe, Funktionsanaloge und Varianten gemäß der Erfindung als Äquivalente verstanden werden, wie sie für den internationalen Patentrechtsharmonisierungsvertrag definiert wurden, sind diese auch ausreichend klar. Die Ausdrücke haben daher eine allgemein anerkannte Bedeutung, so dass sie nicht durch präzisere Angaben ersetzt werden müssen. Der Begriff "im wesentlichen" wird gemäß der genannten Definition in dem internationalen Patentrechtsharmonisierungsvertrag zugelassen, weil er als "Weichzeichner" (Schickedanz, "Die Formulierung von Patentansprüchen", München 2000) von zu scharf bzw. eng definierten Ansprüchen dient. Durch den Begriff "im wesentlichen" soll vermieden werden, dass eine Nukleinsäure, die eine Variante der erfindungsgemäßen Sequenzen SEQ ID Nr. 1 bis SEQ ID Nr. 132 darstellt und dieselbe Funktion auf demselben Weg mit demselben Ergebnis hervorbringt (insbesondere den Nachweis der bevorzugten Pflanzenpathogene) nicht mehr durch den Patentanspruch erfasst wird, wenn sie beispielsweise nicht exakt das identische Ergebnis hervorbringt oder wenn für derartige Homologe, Funktionsanaloge und Varianten, sofern sie als Primer verwendet werden, modifizierte Bedingungen - wie z. B. leicht veränderte pH-Werte von Puffern oder ähnlichem - eingesetzt werden. Auf dem Gebiet der Molekularbiologie sind daher Begriffe wie "funktionsanalog", "homolog", "Varianten" oder "im wesentlichen" ähnlich klar wie Begriffe "mindestens" oder "oberhalb" in der Mechanik. Der Begriff "auf im wesentlichen demselben Weg" bedeutet daher im Sinne der Erfindung, dass die Funktionsanalogen, Homologen und Varianten in einer PCR-Reaktion, bevorzugt in einer Realtime-PCR-Reaktion zur Detektion von Salatfäule-Pathogenen, besonders bevorzugt von Rhizoctonia solani AG1-IB verwendet werden können. Dem Fachmann sind mehrere Wege der Realtime-PCR-Reaktion bekannt, die er als im wesentlichen dieselben Wege bezeichnen würde. Das im wesentlichen selbe Ergebnis liegt immer dann vor, wenn eine Detektion von bevorzugt Rhizoctonia solani AG1-IB so gelingt, dass diese Salatfäule-Pathogene nachgewiesen werden können. In im wesentlichen dieselbe Funktion bedeutet im Sinne der Erfindung, dass der Fachmann - ohne selbst erfinderisch tätig werden zu müssen - insbesondere aus der erfindungsgemäßen Information der Bruchstücke oder deren komplementären Sequenzen ausgewählt aus der Gruppe umfassend aus

SEQ ID Nr. 1 (=1)

GGTGAGGTCACATGATAAGAAAGGTTGTCCTATTGACTCGGAAAAAATACTATAATTAT-
GAGAAAAATGAGGGGTTTCAGGTGATGTCTATGACATGGCACCAGAACCATTGTCACCC-
CAGTTGTGCAAAACTGCTCCCACCCTTTCTCAAGCATGTTGAGTATGGGTGATTCATCA-
TAGGTCTTTATATTGCAAAACAGTTGGTTCAAACCATGTGTTTCAATGATTCAGGA-
TATCTGCTCATCAACACTAGGAATGTCATGTGGCAAGATATGCAGGTCCATTCTCAA-
CAATTGGCTCAATCATTCTGCTAACGTTGGTGACAACACTGGGTTTATATTGAAAACAA-
CATGGAACAACTGTTCCAAATCATATTTGACAAATTTCATATTTATACAAGATGCCAAC-

CAACTCAGCTCCTGAACGTTTGAGACAAGGTTTACACATGATTTGGTTTCAGCAATCTGC-
CAAGCTCCAGATTTGCATGGCTGTGTAGGGGGGTGGGGGAGCACAGCATCCCC-
CAGTGGCTGTGATATACTATGTAAATGGATATGCGTGGATATATCTATATATACCTGGA-
CATGATATATATGGTAATAACCCTTCCCAAAGTGGGTTATGACACTAGAGTAGGTGGTAT-
CAACAAGAGGTAATTGACCTGGGTGTACCCATGGTCAAGAGACCTCCTATATATTGTACA-
GAGGCAGTGTTATGTGTAATTGTATATGAAAAACTATGACCAATAAGAACCCTGCT-
CAGTCTGCAGCCTTACTCATGTAGTGTTGTAGACGTCATGCACAGAGGTGGCACTTA-
TAAGGCCGGTCTATACTACTACACATGGAGTCTCTATGCTACTCTAACTACAATCAGG-
GAGGGGTTGGAGGCAATGCTGGGAGTGACTATGTTAGCACGCTCAACCATACTGCAGCT-
GACTCAGCTATCTGTCGTTGGCCTTGGGCTGCTATGAAGTGTAGAGTGGTGACAAAGAGG-
TAAAAGACCTGGGGATTACCATGGTTGATTGCCCTCCTTATATATTCTAACAAGGCAGTG-
TAATTACATATACAATATCAAATTCTGTACCCAATAAGAACCCCGTTCAGCCTGTGGCCT-
TACTCATCCTGTGACATGGAGGCTTGGCACTGACTGAGGGGATGTGACTAAGGTGGTG-
GAGGCTGACCAATAAGTGCTGGGCTGTTGGTAAGCACTGGGGGTTATGTAACAAGCACT-
GAATTCATGTGACTAAAAAACTAAAGCTAAGGTGTTTTGTTTAAATTTTGAGAAAATAG-
GAATAAATCCTTTGGTGTGTGTGTCATGCCTACAACATGTAGTGACATGGAGGTCTTGG-
CACTGACTGATGGGGATGTGACTTTGGTGCTTCTGCTTGTTATTGCTGATGTAA-
TAAGTGCCAAGGTCACATGATTCAAAAAGTTGTTCTATTGACTTGGAAAAATACTA-
TAGCTTGAGTGCCACCTCTAGGCAGCAAAGACCTTGCTCAGAGGTGGGGTGCTG-
TATGTGTTTGCTTGTTCAATGTATATTGACCTCACCA

2:

GGTGAGGTCACA

3:

TGATAAGAAAGG

4:

TTGTCCTATTGA

5:

CTCGGAAAAAAT

6:

ACTATAATTATG

7:

AGAAAAATGAGG

8:

GGTTTCAGGTGA

9:

TGTCTATGACAT

10:

GGCACCAGAACC

11:

ATTGTCACCCCA

12:

GTTGTGCAAAAC

13:

TGCTCCCACCCT

14:

TTCTCAAGCATG

15:

TTGAGTATGGGT

16:

GATTCATCATAG

17:

GTCTTTATATTG

18:

CAAAACAGTTGG

19:

TTCAAACCATGT

20:

GTTTCAATGATT

21:

CAGGATATCTGC

22:

TCATCAACACTA

23:

GGAATGTCATGT

24:

GGCAAGATATGC

25:

AGGTCCATTCTC

26:

AACAATTGGCTC

27:

AATCATTCTGCT

28:

AACGTTGGTGAC

29:

AACACTGGGTTT

30:

ATATTGAAAACA

31:

ACATGGAACAAC

32:

TGTTCCAAATCA

33:

TATTTGACAAAT

34:

TTCATATTTATA

35:

CAAGATGCCAAC

36:

CAACTCAGCTCC

37:

TGAACGTTTGAG

38:

ACAAGGTTTACA

39:

CATGATTTGGTT

40:

TCAGCAATCTGC

41:

CAAGCTCCAGAT

42:

TTGCATGGCTGT

43:

GTAGGGGGGTGG

44:

GGGGAGCACAGC

45:

ATCCCCCAGTGG

46:

CTGTGATATACT

47:

ATGTAAATGGAT

48:

ATGCGTGGATAT

49:

ATCTATATATAC

50:

CTGGACATGATA

51:

TATATGGTAATA

52:

ACCCTTCCCAAA

53:

GTGGGTTATGAC

54:

ACTAGAGTAGGT

55:

GGTATCAACAAG

56:

AGGTAATTGACC

57:

TGGGTGTACCCA

58:

TGGTCAAGAGAC

59:

CTCCTATATATT

60:

GTACAGAGGCAG

61:

TGTTATGTGTAA

62:

TTGTATATGAAA

63:

AACTATGACCAA

64:

TAAGAACCCTGC

65:

TCAGTCTGCAGC

66:

CTTACTCATGTA

67:

GTGTTGTAGACG

68:

TCATGCACAGAG

69:

GTGGCACTTATA

70:

AGGCCGGTCTAT

71:

ACTACTACACAT

72:

GGAGTCTCTATG

73:

CTACTCTAACTA

74:

CAATCAGGGAGG

75:

GGTTGGAGGCAA

76:

TGCTGGGAGTGA

77:

CTATGTTAGCAC

78:

GCTCAACCATAC

79:

TGCAGCTGACTC

80:

AGCTATCTGTCG

81:

TTGGCCTTGGGC

82:

TGCTATGAAGTG

83:

TAGAGTGGTGAC

84:

AAAGAGGTAAAA
85:
GACCTGGGGATT
86:
ACCATGGTTGAT
87:
TGCCCTCCTTAT
88:
ATATTCTAACAA
89:
GGCAGTGTAATT
90:
ACATATACAATA
91:
TCAAATTCTGTA
92:
CCCAATAAGAAC
93:
CCCGTTCAGCCT
94:
GTGGCCTTACTC
95:
ATCCTGTGACAT
96:
GGAGGCTTGGCA

97:

CTGACTGAGGGG

98:

ATGTGACTAAGG

99:

TGGTGGAGGCTG

100:

ACCAATAAGTGC

101:

TGGGCTGTTGGT

102:

AAGCACTGGGGG

103:

TTATGTAACAAG

104:

CACTGAATTCAT

105:

GTGACTAAAAAA

106:

CTAAAGCTAAGG

107:

TGTTTTGTTTAA

108:

ATTTTGAGAAAA

109:

TAGGAATAAATC
110:
CTTTGGTGTGTG
111:
TGTCATGCCTAC
112:
AACATGTAGTGA
113:
CATGGAGGTCTT
114:
GGCACTGACTGA
115:
TGGGGATGTGAC
116:
TTTGGTGCTTCT
117:
GCTTGTTATTGC
118:
TGATGTAATAAG
119:
TGCCAAGGTCAC
120:
ATGATTCAAAAA
121:
GTTGTTCTATTG

122:

ACTTGGAAAAAT

123:

ACTATAGCTTGA

124:

GTGCCACCTCTA

125:

GGCAGCAAAGAC

126:

CTTGCTCAGAGG

127:

TGGGGTGCTGTA

128:

TGTGTTTGCTTG

129:

TTCAATGTATAT

130:

TGACCTCACCA

131:

AGCGTGCTAACATAGTCACTC

132:

ACACTAGAGTAGGTGGTATCA

Primer bereitstellen kann, die eine einfache, sichere und effektive Detektion von bevorzugt Rhizoctonia solani AG1-IB erlauben, wobei Forward-Primer ein o. g. Bruchstück ganz oder teilweise umfassen können oder aus diesen bestehen und Reverse-Primer eine zu den Bruchstücken reverse Sequenzabfolge umfassen können.

In einer bevorzugten Ausführungsform der Erfindung ist das Bruchstück ausgewählt aus der Gruppe umfassend SEQ ID Nr. 2 bis SEQ ID Nr. 132, wobei bei der Generierung der Reverse-Primer die revers-komplementären Sequenzen zu den Bruchstücken verwendet werden.

Jedes der genannten Bruchstücke oder der komplementären Sequenzen kann anmeldungsgemäß allein oder in Kombination mit einem weiteren anmeldungsgemäßen Bruchstück eingesetzt werden, um als Primer im Sinne der Erfindung verwendet zu werden.

Die Erfindung betrifft demgemäß auch ausgewählte Bereiche, bevorzugt aus der Sequenz gemäß SEQ ID Nr. 131 und/oder SEQ ID Nr. 132 bzw. deren komplementären Sequenzen, wobei die ausgewählten Teilbereiche eng sind und genügend Abstand zu dem bekannten Bereich haben und wobei weiterhin der ausgewählte Bereich kein willkürlich ausgewählter Ausschnitt ist, sondern einer, der zu einer gezielten Auswahl führt, da durch die Auswahl Primer bereitgestellt werden können, die einen spezifischen Nachweis von Salatfäule-Pathogenen, bevorzugt Rhizoctonia solani Spezies, besonders bevorzugt Rhizoctonia solani AG1-IB, insbesondere in einem einzigen Arbeitsschritt ermöglichen.

In einer weiteren bevorzugten Ausführungsform sind die Bruchstücke ausgewählt aus der Gruppe umfassend SEQ ID Nr. 131 und/oder SEQ ID Nr. 132.

Der Fachmann erkennt, dass die Sequenz AGCGTGCTAACATAGTCACTC eine komplementäre Sequenz aus SEQ ID Nr. 1 ist und die Sequenz ACACTAGAGTAGGTGGTATCA direkt aus der Sequenz SEQ ID Nr. 1 ausgewählt wurde. Dieses Vorgehen entspricht dem Designen von Primern aus einem spezifischen DNA Abschnitt.

Selbstverständlich sind dem Fachmann Varianten bekannt, Äquivalente zu den beanspruchten Nukleinsäuremolekülen bzw. deren komplementären Sequenzen (= komplementäre Nukleinsäuremoleküle) herzustellen, die von der Struktur her von den anmeldungsgemäßen im wörtlichen Sinne verschieden sind, aber die gleiche Funktion aufweisen, d. h. funktionsanalog sind. In diesem Sinne sind auch alle Nukleinsäuremoleküle beansprucht, die mindestens 60%, vorzugsweise 70%, bevorzugt 80%, ganz besonders bevorzugt 90% Homologie zu einem Nukleinsäuremolekül gemäß SEQ ID Nr. 1 oder deren komplementären Nukleotidsequenzen aufweisen. Anmeldungsgemäß sind also nicht zahlreiche Varianten und Modifikationen der erfindungsgemäßen Nukleotidsequenz beansprucht, sondern nur diese, die durch den Fachmann durch Routineversuche in Abwandlung von den erfindungsgemäßen generiert werden können und funktionsanalog zur Sequenz SEQ ID Nr. 1 bzw. zu den Sequenzen SEQ ID Nr. 2 bis SEQ ID Nr. 132 sind.

Bei der Generierung der Primer wird aus der Sequenz SEQ ID Nr. 1 ein Bruchstück nach bestimmten Kriterien (Länge an Basenpaaren, hier 21, G/C Verhältnis) ausgewählt. Dann wird geprüft, ob sich der generierte Primer zum Nachweis nur für die AG 1-IB eignet. Die Gesamtsequenz kann auch Homologien (Übereinstimmungen) zu Sequenzen in anderen Organismen aufweisen, wie z.B. im Homo sapiens, der Maus oder andere Organismen.

In einer besonders bevorzugten Ausführungsform der Erfindung ist das Nukleinsäuremolekül eine genomische, eine cDNA oder eine RNA.

Die Erfindung betrifft gemäß den oben gemachten Ausführungen auch die Verwendung der Nukleinsäuresequenzen als Primer, bevorzugt als SCAR-Primer. Ein Primer im Sinne der Erfindung ist ein RNA-Oligonukleotid. Hergestellt werden Primer erfindungsgemäß von dem Enzym Primase aus einzelnen Nukleotiden. Sie werden von DNA-replizierenden Enzymen wie der DNA-Polymerase als Startpunkt benötigt. Sie sind im Sinne der Erfindung kurz genug, um sich spontan an einen vorhandene komplementären DNA-Strang anzulagern, um so die Replikation zu ermöglichen. Die Realtime-PCR ist eine Vervielfältigungsmethode für Nukleinsäuren, die auf dem Prinzip der an sich bekannten PCR beruht und zusätzlich die Möglichkeit der Quantifizierung bietet. Die Quantifizierung wird insbesondere mithilfe von Fluoreszenzmessungen am Ende bzw. während des PCR-Zyklus durchgeführt und unterscheidet sich somit von anderen quantitativen PCR-Methoden, die erst nach Ablauf der PCR ausgewertet werden können, wie beispielsweise die kompetitive PCR. Die Fluoreszenz nimmt hierbei proportional mit der Menge der PCR-Produkte zu, was vorteilhafterweise eine Quantifizierung möglich macht. Damit ist eine gel-elektrophoretische Auftrennung der Fragmente nicht nötig, die Daten sind sofort verfügbar und das Kontaminationsrisiko ist gering.

In einer bevorzugten Ausführungsform der Erfindung wird der Primer zur Bestimmung von Pflanzen-Pathogenen verwendet, insbesondere Gemüsepflanzen-Pathogenen, ganz besonders für Salatpflanzen-, Kartoffelpflanzen-, Zuckerrüben-, Reispflanzen-und/oder Sojabohnenpflanzen-Pathogenen. Bei den Pflanzen-Pathogenen handelt es sich insbesondere um Pilze oder um andere Mikroorganismen; im Sinne der Erfindung können die Pflanzen-Pathogene jedoch auch Viren sein. Wenn die Pflanzen-Pathogene Bakterien sind, kann es sich beispielsweise handeln um: Agrubacterium, Burkholderia, Pseudomonas, Xanthomonas, Xylella, Erwinia, Clavibacter, Rhodococcus, Streptomyces, Spiroplasma bzw. um Rickettsien-ähnliche Organismen oder um Mykoplasmen-ähnliche Organismen. Bevorzugte Oomycota im Sinne der Erfindung sind: Phytophtora cinnamomi, P. lateralis, P. quercina, P. cambivora, P. citricola, P. cactorum, P. infestans, Phythium ultimum, Plasmopara viticola, Bremia lactucae und/oder Pseudoperonospora humuli. Bevorzugte Krankheitserreger, wenn diese Pilze sind, sind im Sinne der Erfindung: Chytridiomycota, Zygomycota, Ascomyceten, Basidomyceten und/oder Deuteromyceten.

Die Erfindung betrifft auch einen Kit, der den Primer ggf. zusammen mit einer Information zum Kombinieren der Inhalte des Kits umfasst. Dieser Kit kann insbesondere zum Bestimmen von Gemüsepflanzen-Pathogenen, insbesondere von Salatfäule-Erregern verwendet werden.

Die Erfindung betrifft auch ein Verfahren zum Bestimmen von Pflanzen-Pathogenen, besonders Gemüsepflanzen-Pathogenen, ganz besonders von Salatpflanzen-, Kartoffelpflanzen-, Zuckerrüben-, Reispflanzen- und/oder Sojabohnenpflanzen-Pathogenen. All diese Pathogene können bevorzugt mit dem erfindungsgemäßen Primer nachgewiesen werden.

In einer bevorzugten Ausführungsform der Erfindung sind die Salatpflanzen ausgewählt aus der Gruppe umfassend: Bataviasalat, Brunnenkresse, Chicorée, Chinakohl, Eisbergsalat, Eichblattsalat, Endiviensalat, Friséesalat, Feldsalat, Gartenkresse, Kopfsalat, Pflücksalat, Radicchiosalat und/oder Römischer Salat. Demgemäß betrifft die Erfindung auch ein Verfahren zur Detektion von Salatfäule-Pathogenen, bei denen die erfindungsgemäßen Primer in einer Realtime-PCR eingesetzt werden. In einer ganz besonders bevorzugten Ausführungsform der Erfindung ist das Salatfäule-Pathogen ausgewählt aus der Gruppe der *Rhizoctonia solani Spezies;* ganz besonders bevorzugt ist die *Rhizoctonia solani* Spezies *R. solani* AG1-IB. Bevorzugt kann demgemäß das erfindungsgemäße Verfahren eingesetzt werden, um direkt aus genomischer DNA aus einer Pflanzen- und/oder Bodenprobe *Rhizoctonia solani -* bevorzugt AG1-IB - nachzuweisen.

In einer bevorzugten Ausführungsform der Erfindung wird die Bodenprobe in einem radialen oder linearen Probenentnahmedesign entnommen, wobei pro Probenentnahme 1 bis 50, bevorzugt 5 bis 30, besonders bevorzugt 7 bis 14 Proben pro Standort entnommen werden. Ein günstiger Zeitpunkt zur Entnahme einer Bodenprobe liegt beispielsweise zwischen November und März. Sie kann bevorzugt mindestens 4 Wochen vor einer ersten Düngung entnommen werden und im Abstand von 6 Monaten, spätestens 3 Jahren wiederholt werden. Am besten eignen sich hierzu Handprobennehmer, die dem Fachmann bekannt sind, oder kurze Bohrstöcke. Für eine Probe sind im Sinne der Erfindung auf der gesamten Fläche gleichmäßig verteilt mindestens 10 bis 15 Einstiche vorzunehmen, wobei die Einstichtiefe 0 bis 100 cm betragen kann. Der Boden aus 10 bis 15 Einstichen kann bevorzugt gemischt werden, wobei von dieser Mischung beispielsweise 500 g in einen sauberen Probenbehälter abgepackt werden. Prinzipiell hat die Festlegung der Probenentnahmestrategie einschließlich der Auswahl der Probenentnahmestandorte, der Festlegung des Probenentnahmedesigns, der Berücksichtigung der Probenentnahmetiefe, der Verwendung geeigneter Probenentnahmegeräte etc. in Abhängigkeit von der jeweiligen Fragestellung zu erfolgen. Als Probenentnahmedesign zur Ermittlung der Pflanzen-Pathogene im Boden wird in potentiellen Belastungsräumen beispielsweise das radiale Probenentnahmedesign eingesetzt. Nach diesem Muster wird die Probenentnahme kreisförmig um einen Mittelpunkt an 1 bis 50 Punkten im Abstand von 1 bis 15, bevorzugt 2 bis 5, ganz besonders bevorzugt 3 m um einen Kreisumfang vorgenommen. Wird im Zuge der Probenentnahme auch eine bodenkundliche Kartierung durchgeführt, so wird diese ebenfalls an dem Mittelpunkt durchgeführt, wo auch die letzte Probe entnommen wurde. Insgesamt können so pro Standort ganz besonders bevorzugt 7 bis 14 Proben entnommen werden, die beispielsweise in einer braunen Weithalsflasche zu einer Probe vereinigt und anschließend zur Analyse und zur Realtime-PCR in ein geeignetes Labor verbracht werden. An Standorten, an denen Inhomogenitäten innerhalb zu beprobender Flächen kein einheitliches Belastungsbild erwarten lassen, wird anstelle des kreisförmigen bevorzugt das lineare Probenentnahmedesign angewendet. Selbstverständlich ist es auch hier möglich, an solchen Standorten das radiale Probenentnahmedesign einzusetzen. Der Arbeitsablauf für die gezielte Entnahme von Bodenproben lässt sich beispielsweise wie folgt skizzieren:
(i) Erkundung und ggf. fakultative Dokumentation der bodenkundlichen Standortverhältnisse und
(ii) unter Berücksichtigung von Vorinformationen sowie unter Einbeziehung der Ergebnisse der Geländebegehung vor Ort Festlegung der Probenentnahmestrategie durch (a) Festlegung des Probenentnahmedesigns, (b) Beprobung in geeigneten Probenentnahmetiefen ggf. unter Berücksichtigung der nationalen gesetzlichen Vorschriften und Gesetze, z. B. Grünland 1 bis 10 cm oder Acker 0 bis 30 cm.

Bevorzugt beträgt die Probenentnahmetiefe 0 bis 100 cm, bevorzugt 0 bis 50 cm, ganz besonders bevorzugt 0 bis 30 cm. Weiterhin ist vorgesehen, dass die Probenentnahmetiefe zwischen Grünland und Ackerfläche differieren kann, so liegt sie beispielsweise im Grünlandbereich bei 0 bis 10 cm und auf Ackerflächen bei 0 bis 30 cm.

In einer weiteren bevorzugten Ausführungsform der Erfindung erfolgt die Probenentnahme mit einem Edelstahl-Probenstecher und oder die Verfüllung der Proben in eine braune Weithalsflasche. Selbstverständlich ist es nicht zwingend erforderlich, die Proben in braune Weithalsflaschen zu verbringen; diese stellen lediglich eine bevorzugte Ausführungsform der Erfindung dar. Die bevorzugten Edelstahl-Probenstecher sind hierbei so ausgebildet, dass sie ein Mindestgehalt von 50 bis 200 Kubikzentimeter, besonders bevorzugt von 100 Kubikzentimeter aufweisen.

Bevorzugt kann das erfindungsgemäße Verfahren so durchgeführt werden, dass die Probenentnahme in vertikaler oder horizontaler Richtung erfolgt. Die Entnahme in vertikaler Richtung hat besonders Bedeutung für die Ermittlung wenn beispielsweise auch die Wasserdurchlässigkeit der Böden untersucht werden soll, oder wenn Substrate mit plattigem Gefüge oder bei schroffen Schichtwechseln entnommen werden. Hierzu wird der Boden bis zur Ansatztiefe der Stechzylinder abgetragen und geglättet. Auf der so entstandenen Stufe werden die Stechzylinder senkrecht eingedrückt oder eingetrieben. Die Entnahme von Stechzylindern in horizontaler Richtung wird beispielsweise angewandt, wenn gleichzeitig Gefüge-bedingte Wasserdurchlässigkeiten untersucht werden sollen. Hierzu werden die Stechzylinder eben in die Wand des Aufschlusses bzw. der Schürfgrube eingedrückt. Generell ist bei der Probenentnahme auf Sauberkeit zu achten. Die Geräte sind nach jeder Probenentnahme sorgfältig zu reinigen, um die Kontaminationsgefahr durch Verschleppung von Pflanzen-Pathogenen auszuschließen. Selbstverständlich muss, nachdem die Probe aus dem Bodenverband gelöst ist, Sorge dafür getragen werden, dass die zu prüfenden Bodenparameter, insbesondere die Pflanzen-Pathogene, sich nicht durch Transport- oder Lagerbedingungen oder Kontakt mit Probenentnahmegeräten verändern. Die Probe kann als gestörte oder ungestörte Probe entnommen werden. Unter einer ungestörten Probe versteht man eine Probe, deren Bodengefüge bei der Entnahme aus dem Boden weitgehend erhalten bleibt. Für die Entnahme von ungestörten Proben eignen sich Stechzylinder, die nach Bodenart, Homogenität und Untersuchungszweck unterschiedliche Größen haben können. Der Mindestgehalt eines Stechzylinders beträgt bevorzugt 100 Kubikzentimeter. Ungestörte Bodenproben sind vorzugsweise bei Feldkapazität im Frühjahr oder Spätherbst zu gewinnen. In Moorböden ist die Entnahme der Proben auch bei geringerem Wassergehalt möglich. Vor der Probenentnahme ist am Profil eine Profilbeschreibung mit ausführlicher Gefügeansprache durchzuführen und zu dokumentieren. Es wird empfohlen, die Probenentnahme von unten nach oben durchzuführen, um sekundäre Kontamination auszuschließen. Ungestörte Proben sind möglichst erschütterungsfrei zu transportieren. Hierzu bieten sich besonders bevorzugt gepolsterte Stechzylinderkisten an, wobei die Proben auch gegen Verdunstung zu sichern sind. Gestörte Proben können Proben sein, bei denen Einzelproben durchmischt werden, wobei aus dieser Mischprobe eine Untersuchungsprobe entnommen wird, die 50 bis 400 g, 100 bis 300 g, bevorzugt 200 g wiegt. Im Folgenden soll die Erfindung anhand eines Beispiels näher erläutert werden, ohne auf dieses Beispiel beschränkt zu sein.

### Beispiel

### Molekulare Detektion von Rhizoctonia solani AG1-IB Isolaten

Die phylogenetischen Beziehungen der Untergruppen von *R. solani* (AG-1) wurden in der vorliegenden Studie auf der Basis von rDNA-ITS Sequenzanalysen ausgewertet. Eine nebenher laufende, zusätzliche "tree analysis" von 40 Sequenzen zeigt, dass die *R. solani* AG-1 Isolate sich in 6 Untergruppen unterteilen lassen, IA, IB, IC, ID, IE und IF. Anschließend wurde eine spezifische und sensible Identifizierungsmethode für *R*. *solani* AG1-IB Isolate mit Hilfe der erfindungsgemäßen Primer entwickelt. Aus einem zufällig ausgewählten vergrößerten polymorphen DNA-Fragment (RAPD) wurde ein molekularer Marker hergestellt, der spezifisch für AG1-IB und -ID Isolate ist. Nach der Umwandlung in einen sequenzcharakterisierten vergrößerten Bereich (SCAR) wurde ein für die Untergruppe AG1-IB spezifisches Primer-Paar (N18-rev/N18-for) zum Gebrauch in einer Polymerase-Kettenreaktion (PCK) entwickelt. Unter optimierten PCR Bedingungen vergrößerte das Primer-Paar ein einzelnes DNA Produkt, welches erfolgreich zur Abgrenzung der Untergruppe IB von den anderen AG1-Untergruppen, unabhängig von ihrem Wirt oder ihrer geographischen Herkunft, eingesetzt werden konnte. Das entwickelte bevorzugte Primer-Paar (AGC GTG CTA ACA THG TCA CTC rev und ACA CTA GAG THG GTG GTA TCA for)kann zum eindeutigen und schnellen Nachweis von *R. solani* AG1-IB in natürlich infiziertem Pflanzengewebe und Proben verschiedener Bodenarten genutzt werden. Die Methode stellt ein leistungsfähiges Instrument für Identifikation und Krankheitsdiagnose dar.

### Bodenbewertung

Zwischen der Quantität bzw. der Inokulumdichte eines Erregers im Boden und der Krankheitsschwere besteht eine positive Korrelation. Daher können auf der Basis der Quantität von *R. solani* AG 1-IB im Boden Aussagen zu Ertragsverlusten der Wirtspflanze, die auf dem Boden angebaut werden sollen, getroffen werden. Ausgehend von diesen Kenntnissen können Entscheidungen zum Anbau einer Wirtspflanze und möglichen Bekämpfungsstrategien erfolgen.
Der Erreger R. solani ist randomisiert im Boden verteilt. Eine ausreichende Menge an Bodenproben sind daher randomisiert von dem zu bewertenden Feld zu sammeln. Die Gesamt-DNA wird mittels Ultra Clean Soil DNA isolation kit (MoBio Laboratories, Solana Beach, California) extrahiert oder einer anderen geigneten Extraktionsmethode.
1) In einer einfachen PCR-Reaktion kann unter Nutzung des spezifischen SCAR Primer Paares *R. solani* AG 1-IB nachgewiesen werden. Die Häufigkeit des Nachweises der AG 1-IB in den Bodenproben steht in Beziehung zum Krankheitsauftreten verursacht durch *R. solani* AG 1-IB. Unter Nutzung der real-time PCR ist zum einen die Empfindlichkeit des Nachweises erhöht und zum anderen können Aussagen zur Quantität des Erregers in den Bodenproben gezogen werden. Eine höhere Quantität korreliert mit einem höheren Krankheitsindex.
2) Es können auch sterilisierte Pflanzensegmente in den Boden eingebracht werden. Die Verteilung sollte ebenfalls randomisiert und in ausreichender Zahl erfolgen. Nach 2-4 Tagen werden die Pflanzenreste wieder entnommen, die Proben kurz mit leitungswasser gespült und die DNA extrahiert (Lee und Taylor 1990) und *R. solani* AG 1-IB in einer PCR-Reaktion mittels des SCAR-Primer Paares ermittelt. Die Häufigkeit des Nachweises korreliert mit dem Krankheitsauftreten. Die Menge des Erregers in den proben, zu bestimmen in einer real-time PCR, korreliert mit der Krankheitsschwere.

Der Pilz *Rhizoctonia solani Kühn* (teleomorph Thanatephorus cucumeris (Frank) Donk) befällt weltweit zahlreiche wichtige landwirtschaftliche und gärtnerische Kulturpflanzen. Eine zunehmende Verbreitung des im Boden lebenden Pathogens *R. sola*ni wird generell in Mitteleuropa beobachtet; dies gilt besonders für eine Fäulniserkrankung, die die unteren Teile von Salatköpfen befällt und in mehreren Regionen Deutschlands verbreitet ist. Laun errechnete allein für Süddeutschland einen wirtschaftlichen Verlust von bis zu 250.000,00 € in der Salatproduktion.

Der Artenkomplex *R. solani* wird in Anastomosengruppen (AGs) unterteilt, und zwar auf der Basis der Häufigkeit der Hyphenanastomose, die zwischen zur selben Gruppe gehörenden Isolaten stattfindet, während Isolate von unterschiedlichen AGs nicht anastomosieren. Bis heute können 13 AGs unterschieden werden. Unterschiede z. B. in der Reichweite des Wirts waren einer der Gründe, einzelne Untergruppen innerhalb der AGs hervorzuheben. Das Anastomose-Konzept wird von Methoden basierend auf pektischen Zymogrammen, Restriktions-Fragment-Länge-Polymorphismen (RFLPs) und DNA Analyse gestützt.

Zahlreiche pflanzenpathogene *R. solani* Isolate gehören zur AG1. Diese Isolate wurden von verschiedenen Wirten entnommen, welche unterschiedliche morphologische Merkmale und verschiedene Grade von Pathogenizität aufwiesen. Aufgrund dieser Unterschiede wurden sie in 4 Untergruppen unterteilt. Isolate der Gruppe AG1-IA verursachen starke Hüllen- und Blattfäule bei Mais, Bohnen- und oberirdische Fäule bei Sojabohnen, während Isolate der Gruppe AG1-IB Netzfäule bei Reis, Bohnen oder Sojabohnen, Wurzelfäule und Fäulnis der unteren Pflanzenteile bei Kohl oder Fäulnis der unteren Pflanzenteile bei der kommerziellen Salatproduktion in mehreren geographischen Regionen bedingt. Krankheiten bei Buchweizen, Karotten, Sojabohnen und Flachs werden von Isolaten der Gruppe AG1-IC verursacht. Isolate der neu unterschiedenen Untergruppe AG1-ID konnten von einer Blattkrankheit der Kaffeepflanze auf den Philippinen isoliert werden. Kuniaga et al. unterschieden die Untergruppen IF (Isolate DR-BV-5, DR-BV-7, DR-BV22, DR-BV1, H32) und IE (Isolat P30) innerhalb der AG-1 auf der Basis einer Sequenzanalyse von rDNA-ITS Sequenzen. Diese Isolate werden mit Netzfäule-Erkrankungen bei gemeinen Bohnen in Zentralamerika und der Karibik in Zusammenhang gebracht.

Vorangegangene Studien haben gezeigt, dass AG1-IB der Verursacher von Fäulniserkrankungen der unteren Pflanzenteile bei Salatpflanzen in verschiedenen Regionen Deutschlands ist. Es wurde jedoch oft mehr als eine AG von einer bestimmten Pflanze isoliert und es kam auch *Botrytis cinerea Pers* im Krankheitskomplex der Fäulnis der unteren Pflanzenteile bei Salatpflanzen vor. Die Symptome der fortgeschrittenen Erkrankung sind bei beiden Pathogenen sehr ähnlich und nicht leicht auseinander zu halten. Die Entwicklung einer schnellen und genauen Methode für die spezifische Identifizierung und den Nachweis von *R. solani* AG1-IB im Krankheitskomplex der Fäulnis der unteren Pflanzenteile ist unerlässlich. Methoden, die auf DNA basieren, scheinen am zuverlässigsten. Die RFLP Analyse von vergrößerten ITS-1, ITS-2 und 5.8S rDNA Regionen zeigte äußert große Abweichungen innerhalb der AG-1 Isolate und es konnten keine Untergruppen der Kategorien von AG 1-IA, -IB und -IC unterschieden werden. Isolate innerhalb *R. solani* AG-1 wurden bis jetzt durch die parallele Analyse von RFLPs, zufällig vergrößerten polymorphen DNAs (RAPD) und Fettsäuren unterschieden. Diese Techniken erlauben Unterscheidungen zwischen und innerhalb von AGs, für die Entwicklung von Methoden zur eindeutigen Diagnose und zum Nachweis von AG-1 Untergruppen in Pflanzen- und Bodenproben wird jedoch die Entwicklung von spezifischen und genauen genetischen Markern benötigt, welche den Nachweis kleinster Mengen von Pilz-DNA möglich machen.

Die Suche nach anonymen Ziel-DNA-Sequenzen ist für das Design von PCR Primern erfolgreich. Die auf PCR basierende RAPD Technik, die einzelne kurze Primer von 10 Nukleotiden-Basen einsetzt, setzt keine Vorinformation über die Zielort-Sequenz voraus und erbrachte den Beweis, eine effiziente Methode zur Identifizierung von molekularen Markern zu sein. Diese Methodologie ermöglicht die Untersuchung einer Vielzahl von Standorten während eines relativ kurzen Zeitraumes, im Gegensatz zur Sequenzanalyse von RFLP Studien. Die Umwandlung einer RAPD Vergrößerung in eine sequenzcharakterisierte Vergrößerungsregion (SCAR) stellt ein spezifisches und sensibles Assay sowohl für das Hervorheben von Resistenzgenen als auch für die Identifizierung von pflanzenpathogenen Pilzen dar. Paran und Michelmore nutzten als erste die Herangehensweise über SCARs, um Resistenzgene des dunklen/falschen Mehltau in Salatpflanzen nachzuweisen. Das SCAR-Primer Design nimmt in der Krankheitsdiagnostik an Popularität zu, da die SCAR-Primer vielseitig, zuverlässig und sensibel sind. Die Vorteile der auf PCR basierenden Diagnoseassays liegen darin, dass das Anlegen einer Kultur der Zielorganismen nicht unbedingt notwendig ist und kleine Mengen von Masken-DNA für eine hohe Reproduktivität genutzt werden können. Die Primer-Konstruktion benötigt allerdings Sequenzdaten von gut charakterisierten (gut unterscheidbaren) Genotypen oder Bakterienstämmen. Das Design der auf diesen Sequenzen basierenden Primer-Paaren kann demzufolge zur eindeutigen Diagnose von Pilzen eingesetzt werden.

Aufgabe der Erfindung war auch die phylogenetische Charakterisierung von AG-1 Isolaten aus verschiedenen geographischen Regionen unter Benutzung von ITS-PCR Technologien und die Entwicklung eines spezifischen molekularen Instruments zur zuverlässigen und sensiblen Identifizierung von *R. solani* AG 1-IB Isolaten.

Die vorliegende Erfindung kann bevorzugt zur Diagnose genutzt werden. Sowohl der Boden, der bepflanzt werden soll, kann untersucht werden, als auch das Saatgut bzw. die Setzlinge. Der Vorteil ist, dass der Boden vor dem Bepflanzen auf das Vorhandensein von *R. solani* geprüft und damit die Bodengesundheit untersucht werden kann. Je nach Ergebnis der Untersuchung kann entweder Salat gepflanzt oder eine Behandlung des Bodens - z.B. mit BCA - angestrebt werden. Der Erfolg der Behandlung kann dann wieder mithilfe des erfindungsgemäßen Verfahrens überprüft werden und eine "Freigabe" des Feldes erfolgen. Ein weiterer Vorteil der Methode ist, dass die Bestimmung von *R. solani* nicht nur qualitativ, sondern auch quantitativ erfolgen kann.

Die anmeldungsgemäße Lehre zeichnet sich durch folgende Merkmale aus:
- Abkehr vom technisch Üblichen
- neue Aufgabenstellung
- Vorliegen eines seit langem ungelösten dringenden Bedürfnisses für die Lösung des mit der Erfindung gelösten Problems
- bisheriges vergebliches Bemühen der Fachwelt
- Einfachheit einer Lösung spricht für erfinderische Tätigkeit, insbesondere da sie kompliziertere Lehren ersetzt
- Entwicklung der wissenschaftlichen Technik ging in eine andere Richtung
- entwicklungsstraffende Leistung
- Fehlvorstellungen der Fachwelt über die Lösung des entsprechenden Problems (Vorurteil)
- technischer Fortschritt, wie z. B.: Verbesserung, Leistungssteigerung, Verbilligung, Ersparnis an Zeit, Material, Arbeitsstufen, Kosten oder schwer beschaffbaren Rohstoffen, erhöhte Zuverlässigkeit, Beseitigung von Fehlern, Qualitätshebung, Wartungsfreiheit, größere Effektivität, höhere Ausbeute, Vermehrung der technischen Möglichkeiten, Bereitstellung eines weiteren Mittels, Eröffnung eines zweiten Weges, Eröffnung eines neuen Gebietes, erstmalige Lösung einer Aufgabe, Reservemittel, Alternativen, Möglichkeit der Rationalisierung, Automatisierung oder Miniaturisierung oder Bereichung des Arzneimittelschatzes
- glücklicher Griff (da aus einer Vielzahl von Möglichkeiten eine bestimmte gewählt wird, deren Ergebnis nicht vorausgesagt werden konnte
- Irrtum in Entgegenhaltungen
- junges Gebiet der Technik
- Kombinationserfindung, d.h. mehrere bekannte Elemente werden zu einer Kombination zusammengeführt, die einen überraschenden Effekt aufweist
- Lizenzvergabe
- Lob der Fachwelt und
- wirtschaftlicher Erfolg.

### Annex zu den Anmeldungsunterlagen - nachgereichtes Sequenzprotokoll

## Patentansprüche

1. Isoliertes Nukleinsäuremolekül ausgewählt aus der Gruppe bestehend aus:
a) einem Nukleinsäuremolekül kodierend eine Nukleinsäuresequenz gemäß SEQ ID Nr. 1, bevorzugt gemäß SEQ ID Nr. 2 bis SEQ ID Nr. 132 oder deren komplementären Nukleotidsequenzen,
b) einem Nukleinsäuremolekül, welches mit einer Nukleinsäuresequenz gemäß a) unter stringenten Bedingungen hybridisiert,
c) einem Nukleinsäuremolekül umfassend eine Nukleotidsequenz, die eine ausreichende Homologie aufweist, um mit einer Nukleotidsequenz gemäß a) oder b) funktionsanalog zu sein,
d) einem Nukleinsäuremolekül, das in Folge des genetischen Codes zu einer Nukleotidsequenz gemäß a) - c) degeneriert ist und
e) einem Nukleinsäuremolekül gemäß einer Nukleotidsequenz nach a) - d), welches durch Deletionen, Additionen, Substitutionen, Translokationen, Inversionen und/oder Insertionen modifiziert und funktionsanalog zu einer Nukleotidsequenz gemäß a) - d) ist bzw. ein Bruchstück einer solchen Sequenz ist,
wobei eine ausreichende Homologie, um funktionsanalog gemäß c) oder funktionsanalog gemäß e) zu sein, bedeutet, dass das funktionsanaloge Nukleinsäuremolekül im wesentlichen dieselbe Funktion auf im wesentlichen demselben Weg und im wesentlichen dasselbe Ergebnis hervorbringt.

2. Nukleinsäuremolekül gemäß Anspruch 1,
**dadurch gekennzeichnet, dass**
das Bruchstück ausgewählt ist aus der Gruppe umfassend
SEQ ID Nr. 2 bis SEQ ID Nr. 132 zur Generierung von Forward-Primern oder den komplementären Sequenzen zur Generierung von Reverse-Primern.

3. Nukleinsäuresequenz nach einem der vorhergehenden Ansprüche,
**dadurch gekennzeichnet, dass**
die Bruchstücke ausgewählt sind aus der Gruppe umfassend
SEQ ID Nr. 131 und/oder SEQ ID Nr. 132.

4. Verwendung der Nukleinsäuresequenzen gemäß einem der Ansprüche 1-3 als Primer, bevorzugt als SCAR-Primer.

5. Verwendung nach dem vorhergehenden Anspruch,
**dadurch gekennzeichnet, dass**
die Sequenz SEQ ID Nr. 131 als Reverse-Primer und die Sequenz SEQ ID Nr. 132 als Forward-Primer eingesetzt werden.

6. Primer umfassend ein Bruchstück gemäß den Nukleinsäuresequenzen nach Anspruch 2 oder 3.

7. Verwendung der Primer nach Anspruch 6 für eine
PCR-Reaktion, bevorzugt eine Realtime-PCR-Reaktion, insbesondere zur Detektion von Salatfäule-Pathogenen, besonders bevorzugt von Rhizoctonia solani AG1-IB.

8. Verwendung der Primer nach Anspruch 7 zur Bestimmung von Pflanzen-Pathogenen, bevorzugt Gemüsepflanzen-Pathogenen, besonders bevorzugt für Salatpflanzen-, Kartoffelpflanzen-, Zuckerrübenpflanzen-, Reispflanzen- und/oder Sojabohnenpflanzen-Pathogene, ganz besonders bevorzugt für Salatfäule-Pathogene, insbesondere ausgewählt aus der Gruppe der Rhizoctonia solani Spezies, wobei bevorzugt die Rhizoctonia solani Spezies Rhizoctonia solani AG1-IB ist.

9. Verwendung nach einem der vorhergehenden Ansprüche 7 oder 8,
**dadurch gekennzeichnet, dass**
der Nachweis von Rhizoctonia solani als direkter Nachweis aus genomischer DNA aus einer Pflanzen- und/oder Bodenprobe erfolgt.

10. Pilzbefall-Detektionskit insbesondere für Salatfäule umfassend mindestens einen Primer gemäß Anspruch 6 und ggf. eine Information zum Kombinieren der Inhalte des Kits.
